# EUROPEAN PATENT APPLICATION

(11) **EP 4 008 324 A1**
(43) Date of publication of application: **08.06.2022**
(21) Application number: 20212264.4
(22) Date of filing: 07.12.2020
(51) Int. Cl.: A61K 31/166, A61K 31/404, A61K 31/519, A61K 31/553, A61K 31/635, A61K 39/00, A61K 45/06, A61P 35/00

(54) **COMBINATIONS COMPRISING AN INHIBITOR OF AN ANTI-APOPTOTIC PROTEIN, SUCH AS BCL-2, BCL-XL, BCLW OR MCL-1, AND A NOTCH SIGNALING PATHWAY INHIBITOR FOR TREATING CANCER**

(71) Applicant: Cellestia Biotech AG, 4057 Basel (CH)
(72) Inventor: LEHAL, Rajwinder, 4057 Basel (CH); URECH, Charlotte, 4057 Basel (CH); VIGOLO, Michele, 4057 Basel (CH)
(74) Representative: Latscha Schöllhorn Partner AG

(57) **Abstract**

The present invention relates to pharmaceutical combinations comprising an inhibitor of an anti-apoptotic protein and a NOTCH signaling pathway inhibitor and their use in a method for the prevention, delay of progression or treatment of cancer in a subject.

## Description

### Field of the invention

The present invention relates to pharmaceutical combinations comprising an inhibitor of an anti-apoptotic protein and a NOTCH signaling pathway inhibitor and their use in a method for the prevention, delay of progression or treatment of cancer in a subject.

### Background of the invention

Despite the ever increasing number of cancer therapies in general, and combination cancer therapies in particular, cancer is still the third most common cause of death worldwide after cardiovascular diseases and infectious/parasitic diseases; in absolute numbers, this corresponds to 7.6 million deaths (ca. 13% of all deaths) in any given year. The WHO estimates deaths due to cancer to increase to 13.1 million by 2030, while the American Cancer Society expects over 1,685,210 new cancer cases diagnosed and 595,690 cancer deaths in the US in 2016. A 2012 survey by McMillan Cancer Support in the UK has revealed that the median survival time of cancer patients overall has increased from 1 year to 6 years since the 1970's. These statistics illustrate the fact that cancer remains a critical health condition and that there is an urgent need for new anticancer drugs.

The rationale for combination chemotherapy in cancer is to use drugs that work by different mechanisms, thereby decreasing the likelihood that resistant cancer cells will develop. On the other hand, administration of two or more drugs to treat a given condition, such as cancer, generally raises a number of potential problems due to complex *in vivo* interactions between drugs. The effects of any single drug are related to its absorption, distribution, and elimination. When two drugs are introduced into the body, each drug can affect the absorption, distribution, and elimination of the other and hence, alter the effects of the other. For instance, one drug may inhibit, activate or induce the production of enzymes involved in a metabolic route of elimination of the other drug. Thus, when two drugs are administered to treat the same condition, it is unpredictable whether each will complement, have no effect on, or interfere with, the therapeutic activity of the other in a subject. Not only may the interaction between two drugs affect the intended therapeutic activity of each drug, but the interaction may increase the levels of toxic metabolites. The interaction may also heighten or lessen the side effects of each drug. Hence, upon administration of two drugs to treat a disease, it is unpredictable what change, either deterioration or improvement, will occur in the side effect profile of each drug. Additionally, it is difficult to accurately predict when the effects of the interaction between the two drugs will become manifest. For example, metabolic interactions between drugs may become apparent upon the initial administration of the second drug, after the two have reached a steady-state concentration or upon discontinuation of one of the drugs. Therefore, the effects of a combination therapy of two or more drugs cannot be easily predicted.

### Summary of the invention

It has now unexpectedly been found that a combination comprising an inhibitor of an anti-apoptotic protein, such as a BCL-2 inhibitor or a MCL-1 inhibitor and a NOTCH signaling pathway inhibitor, such as 6-(4-(tert-butyl)phenoxy)pyridin-3-amine, is useful for the prevention, delay of progression or treatment of cancer, in particular for the prevention, delay of progression or treatment of T-cell acute lymphoblastic leukemia, breast cancer and adenoid cystic carcinoma (ACC). It was unexpectedly found that treatment with said combination provides a synergistic anti-tumor effect above the effect of either agent alone.

Taking these unexpected findings into account, the inventors herewith provide the present invention in its following aspects.

In a first aspect the present invention provides a pharmaceutical combination comprising:
(a) an inhibitor of an anti-apoptotic protein;
(b) a NOTCH signaling pathway inhibitor; and optionally
(c) one or more pharmaceutically acceptable diluents, excipients or carriers.

In a second aspect the present invention provides a pharmaceutical combination as described herein, for use as a medicament.

In a third aspect the present invention provides a pharmaceutical combination as described herein, for use in a method for the prevention, delay of progression or treatment of cancer in a subj ect.

In a fourth aspect the present invention provides kit of parts comprising a first container, a second container and a package insert, wherein the first container comprises at least one dose of a medicament comprising an inhibitor of an anti-apoptotic protein; the second container comprises at least one dose of a medicament comprising a NOTCH signaling pathway inhibitor, and the package insert comprises optionally instructions for treating a subject for cancer using the medicaments.

### Brief description of the figures

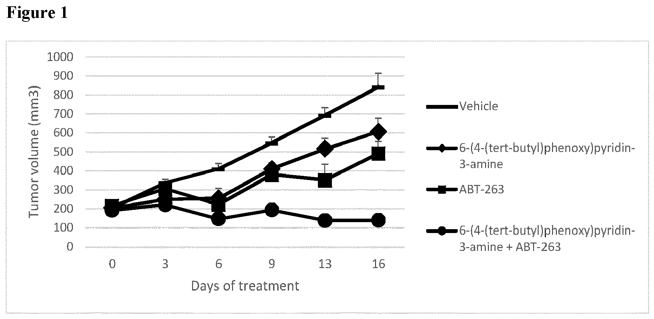

Figure 1: ACC PDX tumor growth in mice treated with vehicle and different drugs. Treatment with a combination of 6-(4-(tert-butyl)phenoxy)pyridin-3-amine and ABT-263 causes regression of ACC tumors.

### Detailed description of the invention

As outlined above, the present invention provides pharmaceutical combinations comprising an inhibitor of an anti-apoptotic protein, such as a BCL-2 inhibitor or a MCL-1 inhibitor and a NOTCH signaling pathway inhibitor, such as 6-(4-(tert-butyl)phenoxy)pyridin-3-amine, which are useful for the prevention, delay of progression, or treatment of cancer.

Thus, in a first aspect the present invention provides a pharmaceutical combination comprising:
(a) an inhibitor of an anti-apoptotic protein;
(b) a NOTCH signaling pathway inhibitor; and optionally
(c) one or more pharmaceutically acceptable diluents, excipients or carriers.

For the purposes of interpreting this specification, the following definitions will apply and whenever appropriate, terms used in the singular will also include the plural and vice versa. It is to be understood that the terminology used herein is for the purpose of describing particular embodiments only and is not intended to be limiting. The terms "comprising", "having", and "including" are to be construed as open-ended terms (i.e., meaning "including, but not limited to,") unless otherwise noted.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "an inhibitor of an anti-apoptotic protein" as used herein refers to a compound capable of blocking the action of proteins that prevent apoptosis mechanisms in cells. Non-limiting examples of inhibitors of an anti-apoptotic protein include B-cell lymphoma 2 (BCL-2) inhibitors, B-cell lymphoma XL (BCL-XL) inhibitors, B-cell lymphoma W (BCLW) inhibitors and Myeloid cell leukemia-1 (MCL-1) inhibitors.

The term "BCL-2 protein family"as used herein refers to the B-cell lymphoma-2 (Bcl-2) family of proteins which regulates apoptosis in cells.

The terms "anti-apoptotic proteins of the BCL-2 family" or "anti-apoptotic BCL-2 family proteins" which are used interchangeably herein refer to members of the B-cell lymphoma-2 (BCL-2) family that prevent apoptosis mechanisms in cells. Non-limiting examples of anti-apoptotic proteins of the BCL-2 family include B-cell lymphoma XL (BCL-XL), B-cell lymphoma W (BCLW), B-cell lymphoma 2 (BCL-2), and Myeloid cell leukemia-1 (MCL-1).

The term "B-cell lymphoma 2 (BCL-2) inhibitor" as used herein refers to a compound capable of blocking the action of anti apoptotic BCL-2 member of the BCL-2 protein family. Non-limiting examples of BCL-2 inhibitors include venetoclax, navitoclax, obatoclax, sabutoclax, ABT-737, PNT-2258, and S-055746.

The term "B-cell lymphoma XL (BCL-XL) inhibitor" as used herein refers to a compound capable of blocking the action of anti apoptotic BCL-XL member of the BCL-2 protein family.

The term "B-cell lymphoma W (BCLW) inhibitor" as used herein refers to a compound capable of blocking the action of anti apoptotic BCLW member of the BCL-2 protein family.

The term "Myeloid cell leukemia-1 (MCL-1) inhibitor" as used herein refers to a compound capable of blocking the action of anti apoptotic MCL-1 member of the BCL-2 protein family. Non-limiting examples of MCL-1 inhibitors include S63845, AMG-176, AMG-397, and AZD5991.

The term "NOTCH signaling pathway inhibitor" as used herein refers to a compound that is inhibiting the NOTCH signalling pathway. NOTCH signaling pathway inhibitor as used herein include a compound of formula (I) as shown below, a γ-secretase inhibitor, a blocking antibody against NOTCH receptors, a blocking antibody against NOTCH ligands, and an inhibitor of NOTCH transcription complex. The NOTCH signalling pathway represents a critical component in the molecular circuits that control cell fate during development, cell survival and cell proliferation (Shih IeM, Wang TL in Cancer Res 2007;67(5):1879-82). Aberrant activation of this pathway contributes to tumorigenesis. The NOTCH family members are being revealed as oncogenes in an ever-increasing number of cancers. The role of NOTCH in human cancer has been highlighted recently by the presence of activating mutations and amplification of *NOTCH* genes in human cancer and by the demonstration that genes/proteins in the NOTCH signalling pathway could be potential therapeutic targets. It has become clear that one of the major therapeutic targets in the NOTCH pathway are the NOTCH receptors, in which γ-secretase inhibitors prevent the generation of the oncogenic (intracellular) domain of NOTCH molecules and suppress the NOTCH activity. Though significant progress has been made in dissecting the complex workings of this signalling pathway, there are very limited options available for developing novel NOTCH inhibitors. However, the pioneering class of NOTCH inhibitors is already in clinical trials for few cancer types, such as γ-secretase inhibitors AL101 from Ayala Pharma (formerly BMS 906024), LY3039478 from Eli Lilly and, PF-03084014 (Nirogacestat) from Springworks Therapeutics, a synthetic small molecule, which inhibits the NOTCH signalling pathway, which may result in induction of growth arrest in tumor cells in which the NOTCH signalling pathway is overactivated.

The term "NOTCH receptors" as used herein refers to the NOTCH receptors NOTCH1, NOTCH2, NOTCH3 and NOTCH4. "A blocking antibody against NOTCH receptors" is a compound specifically binding to the extra-cellular part of the NOTCH receptors hence preventing either constitutive activation of the pathway or activation through ligand binding.

The term "NOTCH ligands" as used herein refers to the NOTCH ligands Delta like 1, Delta like 3, Delta like 4, Jagged, 1, Jagged 2. "A blocking antibody against NOTCH ligands" is a compound specifically binding to one of the ligands hence blocking binding to NOTCH receptors and preventing subsequent activation of the pathway.

The term "inhibitor of NOTCH transcription complex" as used herein refers to a compound that prevents components of NOTCH transcription complex from assembling properly into a functional complex. Non-limiting examples of inhibitors of NOTCH transcription complex include compounds of formula I such as 6-(4-tert-butylphenoxy)pyridin-3-amine; and compounds such as 2-(2-fluorophenoxy)-4-(1-methyl-1*H*-pyrazol-5-yl)benzamide and 2-[2-Methoxy-4-[(4-oxo-2-thioxo-5-thiazolidinylidene)methyl]phenoxy]-acetic acid ethyl ester.

The term "gamma secretase inhibitor" (GSI) as used herein refers to a compound that blocks activity of the gamma secretase complex of proteins.Non-limiting examples of gamma secretase inhibitors include AL-101, AL-102, LY3039478, RO4929097, MK-0752, and PF-03084014.

The terms "individual," "subject" or "patient" are used herein interchangeably. In certain embodiments, the subject is a mammal. Mammals include, but are not limited to primates (including human and non-human primates). In a preferred embodiment, the subject is a human.

The terms "cancer" and "cancerous" as used herein refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, cancer of the gastrointestinal (GI) tract, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

The term "metastatic cancer" as used herein means the state of cancer, e.g. the state of lung cancer or cancer of the gastrointestinal (GI) tract where the cancer cells are transmitted from the original site to one or more sites elsewhere in the body, by the blood vessels or lymphatics, to form one or more secondary tumors at one or more sites or organs besides the original site or organ.

The term "solid tumor" or "solid tumor indication" used herein refers to an abnormal mass of tissue that usually does not contain cysts or liquid areas. Solid tumors may be benign (not cancer), or malignant (cancer). Preferably malignant solid tumors are treated with the methods of the present invention. Different types of malignant solid tumors are usually named for the type of cells that form them. Examples of malignant solid tumors are sarcomas, carcinomas, and lymphomas. Leukemias (cancers of the blood) generally do not form malignant solid tumors (definition according to the national cancer institute of the NIH). Malignant solid tumors include, but are not limited to, abnormal mass of cells which may stem from different tissue types.

The term "hematologic malignancies" used herein refers to cancers that affect the blood, bone marrow, and lymph nodes. Hematologic malignancies are treated with the methods of the present invention. This classification includes but are not limited to various types of leukemia (acute lymphocytic leukemie, chronic lymphocytic leukemia, acute myeloid leukemia, chronic myeloid leukemia), myeloma, and lymphoma (Hodgkin's and non-Hodgkin's).

The term "objective response rate" (ORR) as used herein refers to the proportion of patients with tumor size reduction of a predefined amount and for a minimum time period. Response duration usually is measured from the time of initial response until documented tumor progression. Generally, the FDA has defined ORR as the sum of partial responses plus complete responses. When defined in this manner, ORR is a direct measure of drug antitumor activity, which can be evaluated in a single-arm study. The ORR refers to the sum of complete response (CR) and partial response (PR).

The term "complete response" (CR) as used herein in relation to target lesions refers to disappearance of all target lesions. Any pathological lymph nodes (whether target or non-target) must have reduction in short axis to <10 mm. The term complete response (CR) as used herein in relation to non-target lesions refers to disappearance of all non-target lesions and normalization of tumor marker level. All lymph nodes must be non-pathological in size (<10 mm short axis).

The term "partial response" (PR) as used herein in relation to target lesions refers to at least a 30% decrease in the sum of the diameters of target lesions, taking as reference the baseline sum diameters.

The term "progressive disease" (PD) as used herein in relation to target lesions refers to at least a 20% increase in the sum of the diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progressions. The term progressive disease (PD) as used herein in relation to non-target lesions refers to appearance of one or more new lesions and/or unequivocal progression of existing non-target lesions. Unequivocal progression should not normally trump target lesion status. It must be representative of overall disease status change, not a single lesion increase.

The term "stable disease" (SD) as used herein in relation to target lesions refers to neither sufficient shrinkage to qualify for PR nor sufficient increase to qualify for PD, taking as reference the smallest sum diameters while on study.

The term "progression-free survival" (PFS) as used herein relates to the duration of time from start of treatment to time of progression or death, whichever occurs first.

The term "pharmaceutically acceptable diluents, excipients or carriers" as used herein refers to diluents, excipients or carriers that are suitable for use with humans and/or animals without undue adverse side effects (such as toxicity, irritation, and allergic response) commensurate with a reasonable benefit/risk ratio. "Diluents" are agents which are added to the bulk volume of the active agent making up the solid composition. As a result, the size of the solid composition increases, which makes it easier to handle. Diluents are convenient when the dose of drug per solid composition is low and the solid composition would otherwise be too small. "Excipients" can be binders, lubricants, glidants, coating additives or combinations thereof. Thus, excipients are intended to serve multiple purposes. "Carriers" can be solvents, suspending agents or vehicles, for delivering the instant compounds to a subject.

The term "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e. g. an alkaline metal ion, an alkaline earth metal ion, or an aluminum ion; or coordinates with an organic base such as ethanolamine, diethanolamine, triethanolamine, tromethamine, N-methylglucamine, and the like.

The term "about" as used herein refers to +/- 10% of a given measurement.

Thus, in a first aspect the present invention provides a pharmaceutical combination comprising:
(a) an inhibitor of an anti-apoptotic protein;
(b) a NOTCH signaling pathway inhibitor; and optionally
(c) one or more pharmaceutically acceptable diluents, excipients or carriers.

### Inhibitor of an anti-apoptotic protein

In one embodiment the inhibitor of an anti-apoptotic protein is an inhibitor of an anti-apoptotic protein of the BCL-2 family.

In one embodiment the inhibitor of an anti-apoptotic protein is selected from the group constisting of a B-cell lymphoma 2 (BCL-2) inhibitor, a B-cell lymphoma XL (BCL-XL) inhibitor, a B-cell lymphoma W (BCLW) inhibitor and a Myeloid cell leukemia-1 (MCL-1) inhibitor.

In a preferred embodiment the inhibitor of an anti-apoptotic protein is a B-cell lymphoma 2 (BCL-2) inhibitor or a Myeloid cell leukemia-1 (MCL-1) inhibitor.

In a further preferred embodiment the inhibitor of an anti-apoptotic protein is a B-cell lymphoma 2 (BCL-2) inhibitor.

In a further preferred embodiment the inhibitor of an anti-apoptotic protein a Myeloid cell leukemia-1 (MCL-1) inhibitor.

In a more preferred embodiment the inhibitor of an anti-apoptotic protein is a BCL-2 inhibitor selected from the group consisiting of venetoclax, navitoclax, obatoclax, sabutoclax, ABT-737, PNT-2258, and S-055746, preferably is venetoclax or navitoclax, more preferably is navitoclax.

In a more preferred embodiment the inhibitor of an anti-apoptotic protein is a MCL-1 inhibitor selected from the group consisiting of S63845, AMG-176, AMG-397, and AZD5991, preferably is S63845.

In an even more preferred embodiment the inhibitor of an anti-apoptotic protein is selected from the group consisiting of venetoclax, navitoclax, obatoclax, sabutoclax, ABT-737, PNT-2258, S-055746, S63845, AMG-176, AMG-397, and AZD5991, preferably is venetoclax, navitoclax or S63845, even more preferably is navitoclax or S63845.

Venetoclax which has the chemical name 4-[4-[[2-(4-chlorophenyl)-4,4-dimethylcyclohexen-1-yl]methyl]piperazin-1-yl]-N-[3-nitro-4-(oxan-4-ylmethylamino)phenyl]sulfonyl-2-(1H-pyrrolo[2,3-b]pyridin-5-yloxy)benzamide is described e.g. in WO2005049593 A1 and is represented by the structural formula indicated below:

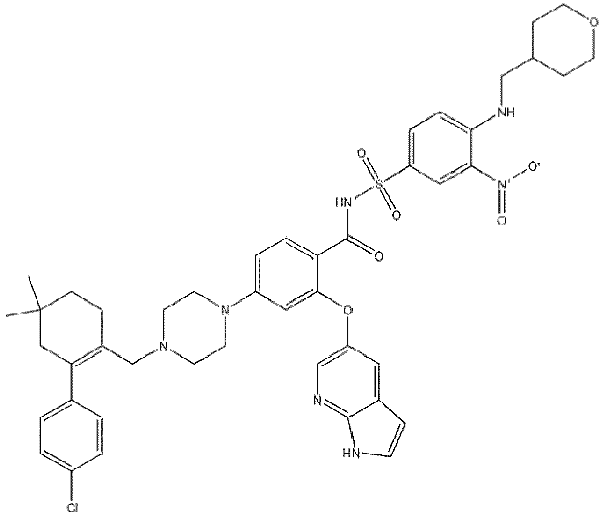

Navitoclax which has the chemical name 4-[4-[[2-(4-chlorophenyl)-5,5-dimethylcyclohexen-1-yl]methyl]piperazin-1-yl]-*N*-[4-[[(2*R*)-4-morpholin-4-yl-1-phenylsulfonylbutan-2-yl]amino]-3-(trifluoromethylsulfonyl)phenyl]sulfonylbenzamide is described e.g. in WO2005049593 A1 and is represented by the structural formula indicated below:

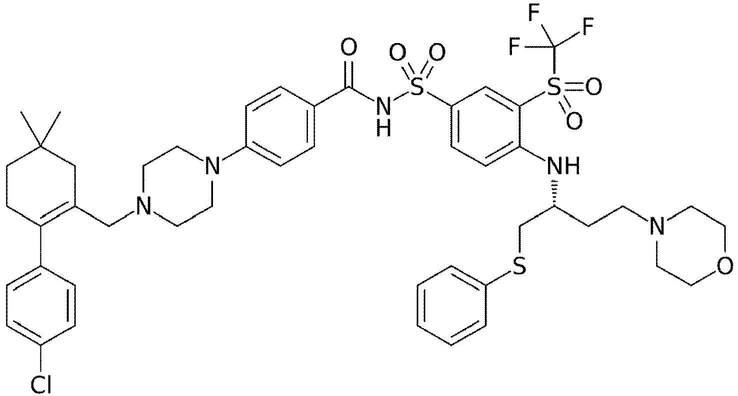

Obatoclax which has the chemical name (2*Z*)-2-[(5*Z*)-5-[(3,5-dimethyl-1*H*-pyrrol-2-yl)methylidene]-4-methoxypyrrol-2-ylidene]indole is described e.g. in US7425553B2 and is represented by the structural formula indicated below:

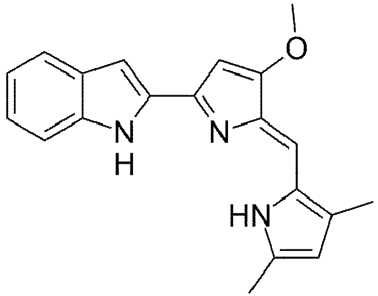

Sabutoclax which has the chemical name 2,3,5-trihydroxy-7-methyl-N-[(2R)-2-phenylpropyl]-6-[1,6,7-trihydroxy-3-methyl-5-[[(2R)-2-phenylpropyl]carbamoyl]naphthalen-2-yl]naphthalene-1-carboxamide is described e.g. in Wei J, et al.. Front Oncol. 2011;1:28 and is represented by the structural formula indicated below:

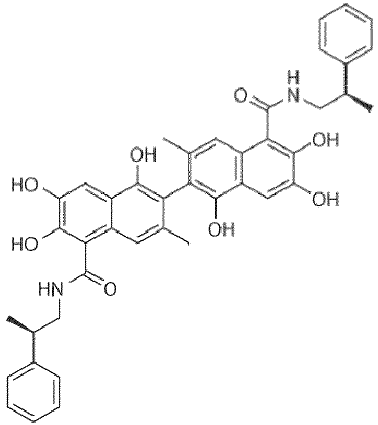

ABT-737 which has the chemical name 4-{4-[(4'-Chlorobiphenyl-2-yl)methyl]piperazin-1-yl}-N-{[4-({(1R)-3-(dimethylamino)-1-[(phenylsulfanyl)methyl]propyl}amino)-3-nitrophenyl]sulfonyl}benzamide is described e.g. in Chauhan D et al. , Oncogene volume 26, 2007, 2374-2380 and is represented by the structural formula indicated below:

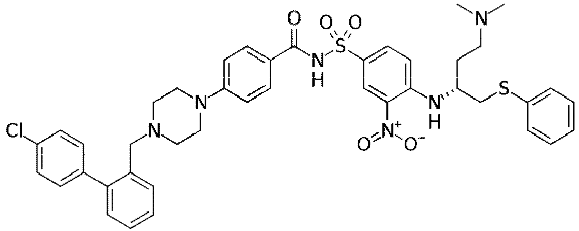

PNT-2258 is a liposomal encapsulated DNA interference (DNAi) oligonucleotide nanoparticle as described e.g. in NCT01733238 or in Ebrahim A. S. Et al., Oncotarget, 2016 Jul 5;7(27).

S-055746 which has the chemical name (S)-N-(4-hydroxyphenyl)-3-(6-(3-(morpholinomethyl)-1,2,3,4-tetrahydroisoquinoline-2-carbonyl)benzo[d][1,3]dioxol-5-yl)-N-phenyl-5,6,7,8-tetrahydroindolizine-1-carboxamide is described e.g. in
Casara et al., Oncotarget . 2018 Apr 13;9(28):20075-20088 or in WO2019161221A2 and is represented by the structural formula indicated below:

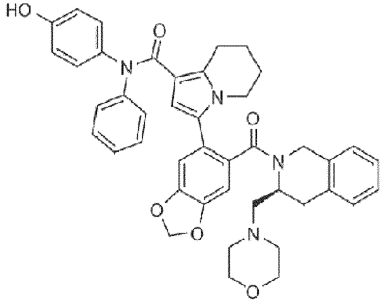

S63845 which has the chemical name (2*R*)-2-[5-[3-chloro-2-methyl-4-[2-(4-methylpiperazin-1-yl)ethoxy]phenyl]-6-(5-fluorofuran-2-yl)thieno[2,3-d]pyrimidin-4-yl]oxy-3-[2-[[2-(2,2,2-trifluoroethyl)pyrazol-3-yl]methoxy]phenyl]propanoic acid is described e.g. in Kotschy A et al., Nature 2016 Oct 27;538(7626) and is represented by the structural formula indicated below:

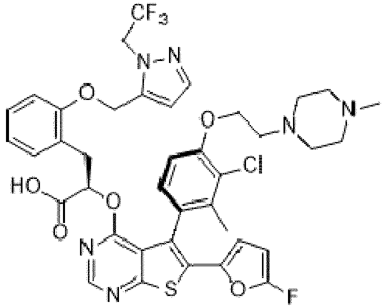

AMG-176 which has the chemical name (3'*R*,4*S*,6'*R*,7'*S*,8'*E*,11'*S*,12'*R*)-7-chloro-7'-methoxy-11',12'-dimethyl-13',13'-dioxospiro[2,3-dihydro-1*H*-naphthalene-4,22'-20-oxa-13λ⁶-thia-1,14-diazatetracyclo[14.7.2.0^{3,6}.0^{19,24}]pentacosa-8,16(25),17,19(24)-tetraene]-15'-one is described e.g. in Caenepeel S et al., Cancer Discov. 2018 Dec;8(12) or in US-2016068545-A1and is represented by the structural formula indicated below:

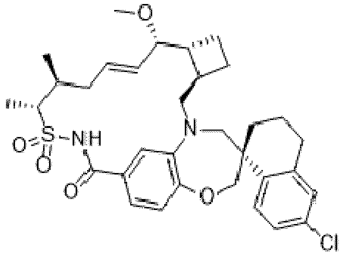

AMG-397 which has the chemical name (13S,31R,32R,4R,5E,8S,9R)-6'-chloro-4-methoxy-8,9-dimethyl-4-{[(9aR)-octahydro-2H-pyrido[1,2-a]pyrazin-2-yl]methyl}-3',4'-dihydro-12H,14H,2'H-spiro[10λ6-thia-11-aza-1(5,7)-[1,5]benzoxazepina-3(1,2)-cyclobutanacyclododecaphan-5-ene-13,1'-naphthalene]-10,10,12-trione is described e.g. in Caenepeel S et al., Proceedings of the Annual Meeting of the American Association for Cancer Research 2020; 2020 Apr 27-28 and Jun 22-24. Philadelphia (PA): AACR; Cancer Res 2020;80(16 Suppl):Abstract nr 6218 or in NCT03465540and is represented by the structural formula indicated below:

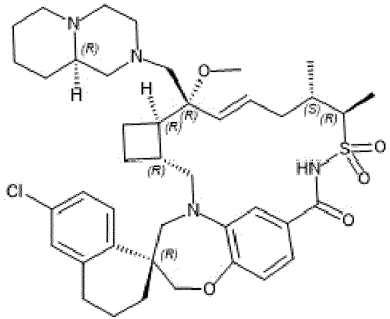

AZD5991 which has the chemical name (Z)-1⁶-chloro-1¹,2¹,2⁵,6¹-tetramethyl-1¹H,2¹H,6¹H-10-oxa-4,8-dithia-1(7,3)-indola-2(4,3),6(3,5)-dipyrazola-9(3,1)-naphthalenacyclotridecaphane-1²-carboxylic acid is described e.g. in CA-3020378-A1 or in Tron A. E. et al., Nat Commun. 2018 Dec 17;9(1)
and is represented by the structural formula indicated below:

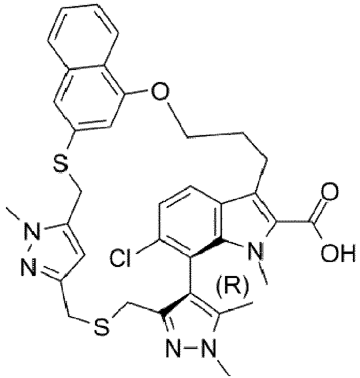

### NOTCH signaling pathway inhibitors

In one embodiment the NOTCH signaling pathway inhibitor is selected from the group consisting of a γ-secretase inhibitor, a blocking antibody against NOTCH receptors, a blocking antibody against NOTCH ligands, and an inhibitor of NOTCH transcription complex.

In one embodiment the NOTCH signaling pathway inhibitor is a compound selected from the group consisting of a γ-secretase inhibitor and an inhibitor of NOTCH transcription complex.

In one embodiment the NOTCH signaling pathway inhibitor is a compound selected from the group consisting of a γ-secretase inhibitor and a compound of formula (I) as shown below. Preferably the γ-secretase inhibitor is selected from the group consisting of AL-101, AL-102, LY3039478, RO4929097, MK-0752, and PF-03084014, more preferably selected from the group consisting of AL-101, AL-102, PF-03084014 and LY3039478.

Thus in a further embodiment the NOTCH signaling pathway inhibitor is a γ-secretase inhibitor selected from the group consisting of AL-101, AL-102, LY3039478, RO4929097, MK-0752, and PF-03084014; or an inhibitor of NOTCH transcription complex.

In a further embodiment the NOTCH signaling pathway inhibitor is a γ-secretase inhibitor; or a compound selected from the group consisting of 2-(2-fluorophenoxy)-4-(1-methyl-1*H-*pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further embodiment the NOTCH signaling pathway inhibitor is a γ-secretase inhibitor selected from the group consisting of AL-101, AL-102, LY3039478, RO4929097, MK-0752, and PF-03084014; or a compound selected from the group consisting of 2-(2-fluorophenoxy)-4-(1-methyl-1*H*-pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further embodiment the NOTCH signaling pathway inhibitor is a compound selected from the group consisting of of 2-(2-fluorophenoxy)-4-(1-methyl-1*H*-pyrazol-5-yl)benzamide, 2-[2-Methoxy-4-[(4-oxo-2-thioxo-5-thiazolidinylidene)methyl]phenoxy]-acetic acid ethyl ester and a compound of formula (I) as shown below.

In a further embodiment the NOTCH signaling pathway inhibitor is a γ-secretase inhibitor selected from the group consisting of AL-101, AL-102, LY3039478, RO4929097, MK-0752, and PF-03084014; or a compound of formula (I) as shown below.

In a preferred embodiment the NOTCH signaling pathway inhibitor is a compound of formula (I) 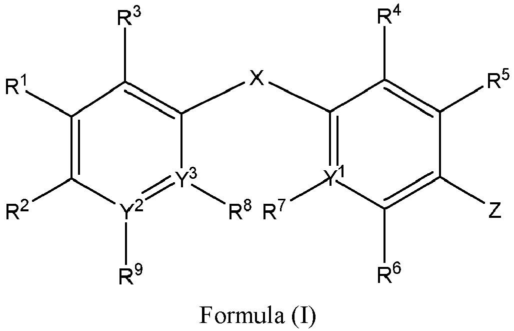 pharmaceutically-acceptable salts, hydrates, solvates, or stereoisomers thereof,
wherein X is selected from CH₂, CF₂, CHF, CO, CHOH, CHO(C₁-C₃) alkyl, NH, N(C₁-C₃ alkyl), S, SO and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl, C₁-C₆ heteroalkyl, C₃-C₁₂ cycloalkyl, C₃-C₁₂ heterocyclyl and C₁-C₆ alkoxy;
wherein R⁴, R⁵ and R⁶ are each independently selected from H, halogen, CN, C₁-C₆ alkoxy, C₁-C₆-S-alkyl, C₁-C₆ alkyl, C₁-C₆ heteroalkyl, C₃-C₁₂ cycloalkyl and C₃-C₁₂ heterocyclyl;
wherein R⁷ is absent when Y¹ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y¹ is C;
wherein R⁸ is absent when Y³ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y³ is C;
wherein R⁹ is absent when Y² is N or is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R¹² is selected from H, NH₂, NHC₁-C₆ alkyl, C₁-C₆ alkyl, C₁-C₆ heteroalkyl, C₂-C₆ alkenyl, C₂-C₆ alkynyl, C₃-C₁₂ cycloalkyl and C₃-C₁₂ heterocyclyl; and optionally one or more pharmaceutically acceptable diluents, excipients or carriers.

The compounds of formula (I) and their synthesis is described in WO2013093885A1 and WO2020208139A1, respectively.

The term "alkyl" as used herein refers to a saturated straight or branched chain group of carbon atoms derived from an alkane by the removal of one hydrogen atom. C₁-C₃ alkyl comprises for example methyl, ethyl, n-propyl, i-propyl and comprises preferably non-branched C₁-C₃ alkyl. C₁-C₄ alkyl comprises for example methyl, ethyl, n-propyl, i-propyl, n-butyl, i-butyl, tert-butyl and comprises preferably non-branched C₁-C₄ alkyl. C₁-C₆ alkyl comprises for example methyl, ethyl, n-propyl, i-propyl, n-butyl, i-butyl, tert-butyl, n-pentyl, and n-hexyl and comprises preferably non-branched C₁-C₆ alkyl. C₁-C₁₀ alkyl comprises for example methyl, ethyl, n-propyl, i-propyl, n-butyl, i-butyl, tert-butyl, n-pentyl, n-hexyl, n-heptyl, n- octyl, n-nonyl or n-decyl and comprises preferably non-branched C₁-C₁₀ alkyl. The term "C₀ alkyl "as used herein refers to a covalent bond. Thus e.g. the term "C₀ alkylOC₀ alkyl aryl" refers to O_{aryl}.

The term "C₀-C₃ alkylOC₀-C₃ alkyl aryl" as used herein refers to Oaryl as defined herein when both C₀-C₃ alkyl groups are C₀ alkyl. The term refers to OC₀-C₃ alkyl aryl when the first C₀-C₃ alkyl group is C₀ alkyl. The term refers to C₀-C₃ alkylOaryl when the second C₀-C₃ alkyl group is C₀ alkyl. Preferably C₀-C₃ alkylOC₀-C₃ alkyl aryl is C₀-C₃ alkylOaryl, more preferably Oaryl or C₁-C₃ alkylOaryl. The term "C₀-C₃ alkylOC₀-C₃ alkyl heteroaryl" as used herein refers to Oheteroaryl as defined herein when both C₀-C₃ alkyl groups are C₀ alkyl. The term refers to OC₀-C₃ alkyl heteroaryl when the first C₀-C₃ alkyl group is C₀ alkyl. The term refers to C₀-C₃ alkylOheteroaryl when the second C₀-C₃ alkyl group is C₀ alkyl. Preferably C₀-C₃ alkylOC₀-C₃ alkyl heteroaryl is C₀-C₃ alkylOheteroaryl, more preferably Oheteroaryl or C₁-C₃ alkylOheteroaryl, most preferably Oheteroaryl. The aryl and the heteroaryl of C₀-C₃ alkylOC₀-C₃ alkyl aryl and C₀-C₃ alkylOC₀-C₃ alkyl heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably are optionally substituted by NH₂.

The term "heteroalkyl" as used herein refers to an alkyl radical as defined herein wherein one, two, three or four hydrogen atoms have been replaced with a substituent independently selected from the group consisting of OR^{a}, C(O)OR^{a}, NR^{b}R^{c}, C(O)NR^{b}R^{c}, S(O)ₙR^{d} (where n is an integer from 0 to 2) and halogen, with the understanding that the point of attachment of the heteroalkyl radical is through a carbon atom, wherein R^{a} is H, C₁-C₃ alkylcarbonyl, C₁-C₃ alkyl, or C₃₋₇ cycloalkyl; R^{b} and R^{c} are each independently H, C₁-C₃ alkylcarbonyl, C₁-C₃ alkyl, C₃₋₇ cycloalkyl or NR^{b}R^{c} is guanidinyl; and when n is 0, R^{d} is H, C₁-C₃ alkyl or C₃₋₇ cycloalkyl, and when n is 1 or 2, R^{d} is C₁-C₃ alkyl or C₃₋₇ cycloalkyl. Preferably. the term "heteroalkyl" or "heteroalkanediyl" as used herein refers to an alkyl radical or an alkanediyl radical as defined herein wherein one, two, three or four hydrogen atoms have been replaced with a substituent independently selected from the group consisting of OH, NH₂, guanidinyl and halogen, more preferably wherein one or two hydrogen atoms have been replaced with a substituent independently selected from the group consisting of OH, NH₂ and halogen. Representative examples include, but are not limited to, 2-hydroxyethyl, 2-hydroxypropyl, 3-hydroxypropyl, 2-hydroxy-1-hydroxymethylethyl, 2-hydroxy-1-methylethyl, 2,3-dihydroxypropyl, 1-hydroxymethylethyl, 3-hydroxybutyl, 2,3-dihydroxybutyl, 1-hydroxy-2-methylpropyl, 3-hydroxy-1-(2-hydroxyethyl)-propyl, 2-hydroxy-1-methylpropyl, 1,1,1-trifluoroethyl, 1,1,1-trifluoromethyl, 2,2,3,3-tetrafluoropropyl.

The term " C₃₋₁₂ cycloalkyl " and "C₃₋₇ cycloalkyl" as used herein refers to a monocyclic, bicyclic, tricyclic or tetracyclic hydrocarbon group, usually to a monovalent saturated monocyclic or bicyclic hydrocarbon group, preferably a monovalent saturated monocyclic goup of 3-12 or 3-7 carbons, respectively derived from a cycloalkane by the removal of a single hydrogen atom. "C₃₋₇ cycloalkyl" includes cyclopropyl, cyclobutyl, cyclopentyl, cyclohexyl and cycloheptyl. The term "C₃₋₁₂ cycloalkyl " and "C₃₋₇ cycloalkyl" as used herein also includes cycloalkyl groups that comprise a C₁₋₃ alkyl radical. Examples of such "C₃₋₇ cycloalkyl" groups comprise cyclopropylmethyl, 2-cyclopropylethyl, cyclobutylmethyl, 2-cyclobutylethyl, cyclopentylmethyl, 2-cyclopentylethyl. Cycloalkyl groups of this invention can be optionally substituted.

The term "aryloxy" or "Oaryl" which are used interchangeably herein refers to a radical -OR where R is an aryl as defined herein, e. g. phenoxy.

The term "C₁-C₆ alkoxy" or "OC₁-C₆ alkyl" which are used interchangeably herein refers to a radical -OR where R is a C₁-C₆ alkyl as defined herein. Examples are methoxy, ethoxy, propoxy, butoxy.

The term "aryl" as used herein refers to a mono- or bicyclic carbocyclic ring system having one or two aromatic rings, and is preferably a monocyclic carbocyclic ring system. The aryl group can also be fused to a cyclohexane, cyclohexene, cyclopentane, or cyclopentene ring or to a cyclohexane, cyclohexene, cyclopentane, or cyclopentene ring comprising a carbonyl group. The aryl groups of this invention can be optionally substituted as further described below. A preferred aryl group and optionally substituted aryl group, respectively of this invention is a phenyl group or substituted phenyl group. Substituents can be e.g. NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹².

The term "heteroaryl" as used herein refers to substituted and unsubstituted aromatic 5-, or 6-membered monocyclic groups and 9- or 10-membered bicyclic groups, preferably a substituted and unsubstituted aromatic 5-, or 6- membered monocyclic group, which have at least one heteroatom (O, S or N) in at least one of the ring(s). Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic group may contain only carbon atoms and may be saturated, partially saturated, or unsaturated. Heteroaryl groups must include at least one fully aromatic ring but the other fused ring or rings may be aromatic or non-aromatic. The heteroaryl group may be attached at any available nitrogen or carbon atom of any ring. Heteroaryl groups of this invention can be optionally substituted as further described below. Usually, a heteroaryl group and optionally substituted heteroaryl group, respectively of this invention is selected from the group consisting of substituted and/or unsubstituted aromatic 5-, or 6- membered monocyclic groups, which have at least one heteroatom (O, S or N), preferably one or two heteroatoms selected from S and N in the ring, more preferably one S and one N in the ring, or one or two N in the ring. A preferred heteroaryl group is an optionally substituted heteroaryl group, selected from the group consisting of an optionally substituted pyridinyl group, an optionally substituted pyrimidinyl group, an optionally substituted di- or triazine group, an optionally substituted thiazole group, an optionally substituted oxazole group, and an optionally substituted imidazole group. An even more preferred heteroaryl group is an optionally substituted pyridinyl group, an optionally substituted pyrimidinyl group, an optionally substituted imidazole group or an optionally substituted thiazole group. Most preferably an optionally substituted pyridinyl group, an optionally substituted imidazole group or an optionally substituted thiazole group, is used as heteroaryl group in the present invention. Optional substituents can be e.g. NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹², or NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl.

The term "heterocyclyl" as used herein means a saturated, monocyclic ring with 3 to 12, preferably with 3 to 7, more preferably 5 to 6 ring atoms which contains up to 3, preferably 1 or 2 heteroatoms selected independently from nitrogen, oxygen or sulfur, and wherein the remaining ring atoms being carbon atoms. Examples of such saturated heterocycles include [1,3]dioxanyl, [1,3]dioxolanyl, pyrrolidinyl, morpholinyl, piperazinyl, piperidinyl, oxazolidinyl, thiazolidinyl, azepanyl and the like. Preferably such heterocyclyl groups are unsubstituted.

The terms "halo" or "halogen" as used herein refers to F, Cl, Br, or I and is preferably F, Cl, or Br, more preferably F.

The term "optionally substituted" or "substituted" means that the referenced group is substituted with one or more additional group(s), preferably with one additional group, individually and independently selected from the listed groups.

AL101 (formerly BMS 906024), which has the chemical name (2R,3S)-N1-((S)-1-methyl-2-oxo-5-phenyl-2,3-dihydro-1H-benzo[e][1,4]diazepin-3-yl)-2,3-bis(3,3,3-trifluoropropyl)succinamide is described e.g. in WO2012129353A1 or NCT03691207 and is represented by the structural formula indicated below:

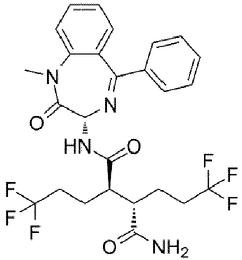

AL102 (BMS 986115), which has the chemical name (2*S*,3*R*)-*N*'-[(3*S*)-5-(3-fluorophenyl)-9-methyl-2-oxo-1,3-dihydro-1,4-benzodiazepin-3-yl]-2,3-bis(3,3,3-trifluoropropyl)butanediamide is described e.g. in WO2014047372A1 and is represented by the structural formula indicated below:

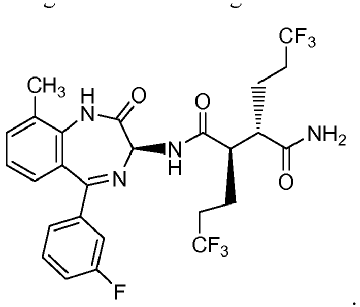

LY3039478 which has the chemical name 4,4,4-trifluoro-*N*-[(2*S*)-1-[[(7*S*)-5-(2-hydroxyethyl)-6-oxo-7*H*-pyrido[2,3-d][3]benzazepin-7-yl]amino]-1-oxopropan-2-yl]butanamide is described e.g. in Massard C et al.. Ann Oncol. 2018;29(9):1911-1917 or in WO2020131998A1 and is represented by the structural formula indicated below:

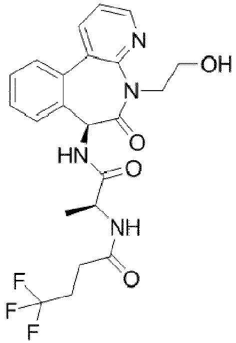

RO4929097 which has the chemical name 2,2-dimethyl-*N*-[(7*S*)-6-oxo-5,7-dihydrobenzo[d][1]benzazepin-7-yl]-*N*'-(2,2,3,3,3-pentafluoropropyl)propanediamide is described e.g. in Huynh C et al. PLoS One. 2011;6(9) or in WO2020131998A1 and is represented by the structural formula indicated below:

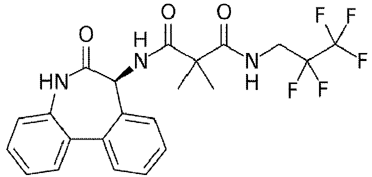

MK-0752 which has the chemical name 3-[4-(4-chlorophenyl)sulfonyl-4-(2,5-difluorophenyl)cyclohexyl]propanoic acidis described e.g. in US2004116404A1 and is represented by the structural formula indicated below:

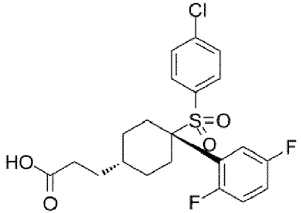

PF-03084014 which has the chemical name (2*S*)-2-[[(2*S*)-6,8-difluoro-1,2,3,4-tetrahydronaphthalen-2-yl]amino]-*N*-[1-[1-(2,2-dimethylpropylamino)-2-methylpropan-2-yl]imidazol-4-yl]pentanamideis described e.g. in US7342118, US7795447 and US7951958 and is represented by the structural formula indicated below:

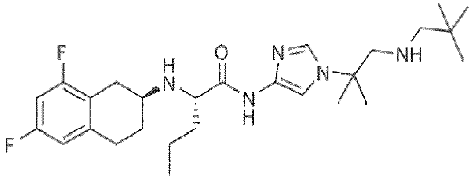

2-(2-fluorophenoxy)-4-(1-methyl-1*H*-pyrazol-5-yl)benzamide also referred to as RBPJ INhibitor1 (RIN1) is described e.g. in Hurtado C et al., 2019 Sci Rep 9, 10811. and denotes a compound according to the Formula:

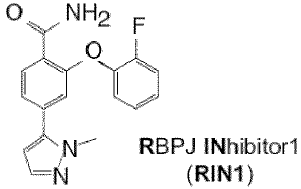

2-[2-Methoxy-4-[(4-oxo-2-thioxo-5-thiazolidinylidene)methyl]phenoxy]-acetic acid ethyl ester ) is obtainable from e.g. Sigma Aldrich and denotes a compound according to the Formula:

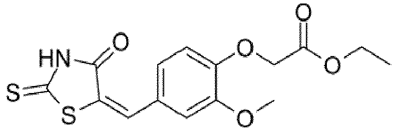

"6-(4-Tert-Butylphenoxy)Pyridin-3-Amine" denotes a compound according to the Formula:

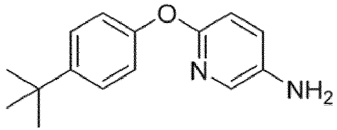

6-(4-Tert-Butylphenoxy)Pyridin-3-Amine is a synthetic small molecule (Molecular Mass: 242.32 g/mol) and is described e.g. in WO2013093885A1.

The present invention also encompasses chemical modifications of the compounds of the present invention to prolong their circulating lifetimes. Non-limiting examples of methods for transiently, or reversibly, pegylating drugs, including polypeptide-based drugs, are provided in U.S. Pat. Nos. 4,935,465 (issued in Jun. 19, 1990) and 6,342,244 (issued Jan. 29, 2002); and in U.S. published applications number US2006/0074024. One skilled in the art would typically find more details about PEG-based reagents in, for example, published applications WO2005047366, US2005171328, and those listed on the NEKTAR PEG Reagent Catalog^{®} 2005-2006 (Nektar Therapeutics, San Carlos, Calif.).

The invention also relates to salts, hydrates or solvates of the compounds of the present invention. Preferably, these salts, hydrates and/or solvates are pharmaceutically acceptable.

The invention also relates to stereoisomers of the compounds of formula (I). "Stereoisomer" or "stereoisomers" refer to compounds that differ in the chirality of one or more stereocentres. Stereoisomers include enantiomers and diastereomers. A compound of formula (I) may exist in stereoisomeric form if they possess one or more asymmetric centres or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (see discussion in Chapter 4 of Advanced Organic Chemistry, 4th ed., J. March, John Wiley and Sons, New York, 1992).

A skilled person will know that, if the compounds of the present invention contain charged group, a suitable counterion will be derived from an organic or inorganic acid. Such counterions include halide (such as chloride, bromide, fluoride, iodide), sulfate, phosphate, acetate, succinate, citrate, lactate, maleate, fumarate, palmitate, cholate, glutamate, glutarate, tartrate, stearate, salicylate, methanesulfonate, benzenesulfonate, sorbate, picrate, benzoate, cinnamate, and the like. If the polar moiety is a negatively charged group, a suitable counterion will be selected from sodium, ammonium, barium, calcium, copper, iron, lithium, potassium and zinc, and the like.

When R¹ is C₀-C₃ alkylOC₀-C₃ alkyl aryl or C₀-C₃ alkylOC₀-C₃ alkyl heteroaryl, the optional substitutions of the aryl and the heteroaryl group are preferably in para position.
When R² is aryl or heteroaryl, the optional substitutions are preferably in ortho or meta position, provided that the substitutents are not halogen, OC₁-C₆ alkyl or methyl and are in para position when the substituents are halogen, OC₁-C₆ alkyl or methyl.
When R⁹ is C₀-C₃ alkylOC₀-C₃ alkyl aryl or C₀-C₃ alkylOC₀-C₃ alkyl heteroaryl the optional substitutions of the aryl and the heteroaryl group are preferably in para position.

In one embodiment X is selected from CH₂, CF₂, CHF, NH, N(C₁-C₃ alkyl), S, SO and O. In a further embodiment X is selected from CO, CHOH, CHO(C₁-C₃) alkyl, S, SO and O. In a preferred embodiment X is selected from CH₂, NH, and O. In a more preferred embodiment X is selected from CH₂, CO, CHOH, CHO(C₁-C₃) alkyl, NH, and O. In an even more preferred embodiment X is selected from CH₂, CO, CHOH, CHO(C₁-C₃) alkyl, and O. In a particular preferred embodiment X is selected from CH₂, CO, CHOH, CHOCH₃, and O. In a more particular preferred embodiment X is selected from CH₂ and O. In an even more particular preferred embodiment X is O.

In one embodiment R¹ is selected from H, halogen, C₁-C₆ alkyl, C₁-C₆ heteroalkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further embodiment R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl. In a preferred embodiment R¹ is selected from H, halogen and C₁-C₄ alkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further preferred embodiment R¹ is selected from H, halogen, C₁-C₆ alkyl and C₁-C₆ heteroalkyl. In a more preferred embodiment R¹ is selected from H, C₁-C₆ alkyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted NH₂; C₀-C₃ alkylOC₀-C₃ alkyl heteroaryl wherein the heteroaryl is optionally substituted by NH₂, C₁-C₆ alkyl, C₁-C₆ heteroalkyl, halogen, CN, preferably is optionally substituted by NH₂. In an even more preferred embodiment R¹ is selected from H, C₁-C₄ alkyl and C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further more preferred embodiment R¹ is selected from H, halogen and C₁-C₄ alkyl. In an even more preferred embodiment R¹ is selected from H, methyl, C₀-C₃ alkylOC₀-C₃ alkyl aryl wherein the aryl is optionally substituted by NH₂, C₁-C₆ alkyl, C₁-C₆ heteroalkyl, halogen, CN, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂. In a further even more preferred embodiment R¹ is selected from H and methyl.

In one embodiment R² is selected from C₁-C₆ alkyl, C₃-C₁₂ cycloalkyl, aryl and heteroaryl wherein the aryl and the heteroaryl is substituted by a substituent selected from NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹² and C₁-C₆ alkyl C(O)R¹². In a further embodiment R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C(O)R¹², C₁-C₆ alkyl C(O)R¹².
In a preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C₃-C₁₂ cycloalkyl, C₃-C₁₂ heterocyclyl, C(O)R¹² and C₁-C₆ alkyl C(O)R¹². In a more preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹². In a further more preferred embodiment R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C₃-C₁₂ cycloalkyl, C₃-C₁₂ heterocyclyl. In a particular preferred embodiment R² is selected from C₁-C₆ alkyl, C₃-C₁₂ cycloalkyl, aryl and heteroaryl wherein the aryl and the heteroaryl are optionally substituted by C₁-C₆ alkyl, halogen, C₁-C₆ heteroalkyl, C(O)R¹², C₁-C₆ alkyl C(O)R¹². In a further particular preferred embodiment R² is selected from C₁-C₆ alkyl, C₃-C₁₂ cycloalkyl, aryl and heteroaryl wherein the aryl and the heteroaryl are optionally substituted by C₁-C₆ alkyl, halogen. In a further particular preferred embodiment R² is selected from C₁-C₆ alkyl, C₃-C₁₂ cycloalkyl, aryl and heteroaryl wherein the aryl and the heteroaryl are optionally substituted by NH₂, halogen, CN. In a further particular preferred embodiment R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹². Among the particular preferred embodiments the embodiment wherein R² is selected from aryl and heteroaryl wherein the aryl and the heteroaryl are optionally substituted by C₁-C₆ alkyl, halogen, C₁-C₆ heteroalkyl, C(O)R¹², C₁-C₆ alkyl C(O)R¹² is preferred. In an even more particular preferred embodiment R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹².
In an even more particular preferred embodiment R² is selected from C₁-C₆ alkyl, C₃-C₁₂ cycloalkyl, phenyl, pyridyl, imidazole and thiazole, wherein the phenyl, pyridyl, imidazole and thiazole each are optionally substituted by NH₂, OC₁-C₆ alkyl, C₁-C₆ alkyl, C₁-C₆ heteroalkyl, halogen, CN, C(O)R¹², C₁-C₆ alkyl C(O)R¹². In a further even more particular preferred embodiment R² is selected from C₁-C₆ alkyl, C₃-C₁₂ cycloalkyl, phenyl, thiazole, pyridyl and imidazole wherein the phenyl, thiazole, pyridyl and imidazole each are optionally substituted by NH₂, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by NH₂, C₁-C₆ alkyl, C₁-C₆ heteroalkyl, halogen, CN, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen, C₁-C₆ heteroalkyl, C(O)R¹², C₁-C₆ alkyl C(O)R¹². In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by NH₂, halogen, CN. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C(O)R¹², C₁-C₆ alkyl C(O)R¹². Among the even more particular preferred embodiments the embodiment wherein R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen, C₁-C₆ heteroalkyl, C(O)R¹², C₁-C₆ alkyl C(O)R¹² is preferred. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each are optionally substituted by C₁-C₆ alkyl, halogen. In a further even more particular preferred embodiment R² is selected from C₁-C₆ alkyl, C₃-C₁₂ cycloalkyl, phenyl, pyridyl, imidazole and thiazole wherein the phenyl, pyridyl, imidazole and thiazole each optionally substituted by C(O)R¹², C₁-C₆ alkyl C(O)R¹². In a further even more particular preferred R² is selected from C₁-C₆ alkyl, C₃-C₁₂ cycloalkyl, phenyl, pyridyl, imidazole and thiazole, wherein the phenyl, pyridyl, imidazole and thiazole each are optionally substituted by OC₁-C₆ alkyl, C₁-C₆ alkyl, C₁-C₆ heteroalkyl, halogen, C₁-C₆ alkyl C(O)R¹². In a most preferred embodiment R² is C₁-C₆ alkyl, more preferably tert-butyl.
In a preferred embodiment when R² is heteroaryl the heteroaryl is an optionally substituted aromatic 5-, or 6- membered monocyclic group.

In one embodiment R³ is selected from H, halogen, C₁-C₆ alkyl, and C₃-C₁₂ cycloalkyl. In a preferred embodiment R³ is selected from H, halogen, C₁-C₄ alkyl, and C₃-C₇ cycloalkyl. In a more preferred embodiment R³ is selected from H, halogen and C₁-C₄ alkyl. In an even more preferred embodiment R³ is H.

In one embodiment R⁴, R⁵ and R⁶ are each independently selected from H, halogen, CN, C₁-C₆ alkyl and C₁-C₆ heteroalkyl. In a preferred embodiment R⁴, R⁵ and R⁶ are each independently selected from H, halogen, CN, C₁-C₄ alkyl and C₁-C₄ heteroalkyl. In an even more preferred embodiment R⁴, R⁵ and R⁶ are each independently selected from H, halogen, and C₁-C₄ alkyl. In a particular preferred embodiment R⁴, R⁵ and R⁶ are each independently selected from H, halogen, and methyl. In an more particular preferred embodiment R⁴ is selected from H and halogen and/or R⁵ and/or R⁶ are selected from H and C₁-C₆ alkyl, in particular from H and C₁-C₄ alkyl, more particular from H and methyl.

In one embodiment R⁷ is absent when Y¹ is N or is selected from H, halogen, C₁-C₆ alkyl and C₃-C₁₂ cycloalkyl when Y¹ is C. In a preferred embodiment R⁷ is absent when Y¹ is N or is selected from H, halogen, C₁-C₄ alkyl and C₃-C₇ cycloalkyl when Y¹ is C. In a more preferred embodiment R⁷ is absent when Y¹ is N or is selected from H, halogen and C₁-C₄ alkyl, preferably selected from H, halogen, and methyl when Y¹ is C. In an even more preferred embodiment R⁷ is absent when Y¹ is N or is selected from H, halogen, and methyl when Y¹ is C. In a particular preferred embodiment R⁷ is absent when Y¹ is N or is selected from H and halogen when Y¹ is C. In a more particular preferred embodiment R⁷ is absent.
In one embodiment R⁸ is absent when Y³ is N or is selected from H, halogen, C₁-C₆ alkyl and C₃-C₁₂ cycloalkyl when Y³ is C. In a preferred embodiment R⁸ is absent when Y³ is N or is selected from H, halogen, C₁-C₄ alkyl and C₃-C₇ cycloalkyl when Y³ is C. In a more preferred embodiment R⁸ is absent when Y³ is N or is selected from H, halogen and C₁-C₄ alkyl preferably selected from H, halogen, and methyl when Y³ is C. In an even more preferred embodiment R⁸ is absent when Y³ is N or is selected from H, halogen, and methyl when Y³ is C. In a particular preferred embodiment R⁸ is absent when Y³ is N or is selected from H and halogen when Y³ is C. In a particular preferred embodiment R⁸ is H.
In one embodiment R⁹ is absent when Y² is N or is selected from H, halogen, C₁-C₆ alkyl and C₃-C₁₂ cycloalkyl when Y² is C. In a preferred embodiment R⁹ is absent when Y² is N or is selected from H, halogen, C₁-C₄ alkyl and C₃-C₇ cycloalkyl when Y² is C. In a more preferred embodiment R⁹ is absent when Y² is N or is selected from H and C₁-C₄ alkyl preferably selected from H, halogen, and methyl when Y² is C. In an even more preferred embodiment R⁹ is absent when Y² is N or is selected from H, halogen, and methyl when Y² is C. In a particular preferred embodiment R⁹ is absent when Y² is N or is selected from H and halogen, preferably H, when Y² is C.

In one embodiment R⁷ is absent when Y¹ is N or is selected from H, halogen, C₁-C₆ alkyl and C₃-C₁₂ cycloalkyl when Y¹ is C, preferably selected from H, halogen, and methyl when Y¹ is C, R⁸ is absent when Y³ is N or is selected from H, halogen, C₁-C₆ alkyl and C₃-C₁₂ cycloalkyl, preferably selected from H, halogen, and methyl when Y³ is C, and R⁹ is absent when Y² is N or is selected from H, halogen, C₁-C₆ alkyl and C₃-C₁₂ cycloalkyl, preferably selected from H, halogen, and methyl when Y² is C.

In a preferred embodiment R⁷ is absent when Y¹ is N or is selected from H and halogen when Y¹ is C, R⁸ is absent when Y³ is N or is H when Y³ is C, and R⁹ is absent when Y² is N or is selected from H and methyl when Y² is C.

In one embodiment at least one of R¹⁰ and R¹¹ is C₁-C₆ alkyl, preferably C₁-C₄ alkyl, more preferably methyl. In a preferred embodiment R¹⁰ and R¹¹ are independently selected from H and methyl. In a more preferred embodiment R¹⁰ is H and R¹¹ is selected from H and C₁-C₄ alkyl. In an even more preferred embodiment R¹⁰ is H and R¹¹ is H or methyl. In a particular preferred embodiment R¹⁰ is H and R¹¹ is C₁-C₆ alkyl. In a more particular preferred embodiment R¹⁰ is H and R¹¹ is C₁-C₄ alkyl. In an even more particular preferred embodiment R¹⁰ is H and R¹¹ is methyl.

In one embodiment R¹² is selected from NH₂, NHC₁-C₆ alkyl, C₁-C₆ alkyl, and C₁-C₆ heteroalkyl. In a preferred embodiment R¹² is selected from NH₂, C₁-C₆ alkyl, and C₁-C₆ heteroalkyl. In a more preferred embodiment R¹² is NH₂.

In one embodiment Y¹ is N. In a preferred embodiment Y¹ and Y² are each independently selected from N and C and Y³ is C. In a more preferred embodiment Y¹ is selected from N and C and Y² and Y³ are selected from N and C with the proviso that one of Y² and Y³ is C. In a particular preferred embodiment Y¹ is N and R⁷ is absent, Y² is selected from N and C and Y³ is C. In a more particular preferred embodiment Y¹ is N and R⁷ is absent.

In one embodiment at least one of the substituents selected from R¹, R³, R⁴, R⁵, R⁶, R⁷, R⁸, and R⁹ is not H. In a further embodiment at least one of the substituents selected from R¹, R³, R⁸, and R⁹ is not H.

Preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl; with the proviso that when R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl
wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, R² is selected from aryl and heteroaryl wherein the aryl and the heteroaryl are optionally substituted by NH₂, halogen, CN, preferably wherein the aryl and the heteroaryl are not substituted.

More preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl, with the proviso that when R² is selected from aryl and heteroaryl wherein the aryl and the heteroaryl are substituted by C₁-C₆ alkyl, halogen, C₁-C₆ heteroalkyl, C(O)R¹², C₁-C₆ alkyl C(O)R¹², R¹ is selected from H, halogen, C₁-C₆ alkyl and C₁-C₆ heteroalkyl, preferably is H or methyl.

Further more preferred compounds of formula (I) pharmaceutically-acceptable salts, hydrates, solvates, or stereoisomers thereof are those, wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more preferred compounds of formula (I) pharmaceutically-acceptable salts, hydrates, solvates, or stereoisomers thereof are those ,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, CN;
wherein R³ is selected from H, halogen and C₁-C₄ alkyl;
wherein R⁴, R⁵ and R⁶ are each independently selected from H, halogen, and C₁-C₄ alkyl; wherein R⁷ is absent when Y¹ is N or is selected from H, halogen and C₁-C₄ alkyl when Y¹ is C; wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Even more preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₄ alkyl and C₁-C₄ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₄ alkyl and C₃-C₇ cycloalkyl;
wherein R⁴, R⁵ and R⁶ are each independently selected from H, halogen, CN, C₁-C₄ alkyl and C₁-C₄ heteroalkyl;
wherein R⁷ is absent when Y¹ is N or is selected from H, halogen, C₁-C₄ alkyl and C₃-C₇ cycloalkyl when Y¹ is C;
wherein R⁸ is absent when Y³ is N or is selected from H, halogen, C₁-C₄ alkyl and C₃-C₇ cycloalkyl when Y³ is C;
wherein R⁹ is absent when Y² is N or is selected from H, halogen, C₁-C₄ alkyl and C₃-C₇ cycloalkyl when Y² is C; and
wherein R¹² is selected from NH₂, NHC₁-C₆ alkyl, C₁-C₆ alkyl, and C₁-C₆ heteroalkyl.

Particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, more preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, C₁-C₆ heteroalkyl, halogen, C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl; wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ heteroalkyl;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, halogen;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, preferably selected from CH₂, CO, CHOH, CHO(C₁-C₃) alkyl, and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂, wherein R¹ is preferably selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN, preferably optionally substituted by halogen, C₁-C₆ alkyl;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl; with the proviso that when R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, R² is selected from aryl and heteroaryl wherein the aryl and the heteroaryl are optionally substituted by NH₂, halogen, CN, preferably wherein the aryl and the heteroaryl are not substituted.

More preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl, with the proviso that when R² is selected from aryl and heteroaryl wherein the aryl and the heteroaryl are substituted by C₁-C₆ alkyl, halogen, C₁-C₆ heteroalkyl, C(O)R¹², C₁-C₆ alkyl C(O)R¹², R¹ is selected from H, halogen, C₁-C₆ alkyl and C₁-C₆ heteroalkyl, preferably is H or methyl.

Further more preferred compounds of formula (I) pharmaceutically-acceptable salts, hydrates, solvates, or stereoisomers thereof are those,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C; and
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more preferred compounds of formula (I) pharmaceutically-acceptable salts, hydrates, solvates, or stereoisomers thereof are those ,
wherein X is selected from CH₂, NH and O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably wherein R¹ is selected from H, halogen, C₁-C₆ alkyl, C₃-C₁₂ cycloalkyl, C₃-C₁₂ heterocyclyl, C₁-C₆ alkoxy; C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, CN;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Even more preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₄ alkyl and C₁-C₄ heteroalkyl;
wherein R² is selected from C₁-C₆ alkyl, C₃-C₁₂ cycloalkyl, aryl and heteroaryl wherein the aryl and the heteroaryl are optionally substituted by NH₂, C₁-C₆ alkyl, C₁-C₆ heteroalkyl, halogen, CN, C(O)R¹², C₁-C₆ alkyl C(O)R¹²;
wherein R³ is selected from H, halogen, C₁-C₄ alkyl and C₃-C₇ cycloalkyl;
wherein R⁴, R⁵ and R⁶ are each independently selected from H, halogen, CN, C₁-C₄ alkyl and C₁-C₄ heteroalkyl;
wherein R⁷ is absent when Y¹ is N or is selected from H, halogen, C₁-C₄ alkyl and C₃-C₇ cycloalkyl when Y¹ is C;
wherein R⁸ is absent when Y³ is N or is selected from H, halogen, C₁-C₄ alkyl and C₃-C₇ cycloalkyl when Y³ is C;
wherein R⁹ is absent when Y² is N or is selected from H, halogen, C₁-C₄ alkyl and C₃-C₇ cycloalkyl when Y² is C; and
wherein R¹² is selected from NH₂, NHC₁-C₆ alkyl, C₁-C₆ alkyl, and C₁-C₆ heteroalkyl.

Particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, more preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, C₁-C₆ heteroalkyl, halogen, C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ heteroalkyl;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C₁-C₆ alkyl, halogen;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from H, halogen and C₁-C₄ alkyl;
wherein R² is selected from C₁-C₆ alkyl, C₃-C₁₂ cycloalkyl, aryl and heteroaryl wherein the aryl and the heteroaryl are optionally substituted by C(O)R¹², C₁-C₆ alkyl C(O)R¹²;
wherein R³ is selected from H, halogen and C₁-C₄ alkyl;
wherein R⁴, R⁵ and R⁶ are each independently selected from H, halogen and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C;
wherein R⁹ is absent when Y² is N or is selected from H, halogen and C₁-C₄ alkyl when Y² is C; and
wherein R¹² is selected from NH₂, NHC₁-C₆ alkyl, C₁-C₆ alkyl, and C₁-C₆ heteroalkyl.

Further particular preferred compounds of formula (I) pharmaceutically-acceptable salts, hydrates, solvates, or stereoisomers thereof are those,
wherein X is selected from CH₂, NH and O, and is preferably O;
wherein Y¹, Y², and Y³ are each independently selected from N and C, preferably Y¹ and Y² are each independently selected from N and C and Y³ is C, more preferably Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl, preferably independently selected from H and methyl, preferably R¹⁰ is H and R¹¹ is H or methyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂, wherein R¹ is preferably selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, preferably is optionally substituted by NH₂;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN, preferably optionally substituted by halogen, C₁-C₆ alkyl;
wherein R³ is selected from H, halogen and C₁-C₄ alkyl;
wherein R⁴, R⁵ and R⁶ are each independently selected from H, halogen, and C₁-C₄ alkyl;
wherein R⁷ is absent when Y¹ is N or is selected from H, halogen and C₁-C₄ alkyl when Y¹ is C;
wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Further particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy and C₁-C₆ heteroalkyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

More particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁷ is absent when Y¹ is N or is selected from H, halogen, C₁-C₆ alkyl and C₃-C₁₂ cycloalkyl when Y¹ is C;
wherein R⁸ is absent when Y³ is N or is selected from H, halogen, C₁-C₆ alkyl and C₃-C₁₂ cycloalkyl when Y³ is C;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl and C₃-C₁₂ cycloalkyl;
wherein R⁴, R⁵ and R⁶ are each independently selected from H, halogen, CN, C₁-C₆ alkyl and C₁-C₆ heteroalkyl;
wherein R⁸ is selected from H, halogen, C₁-C₆ alkyl and C₃-C₁₂ cycloalkyl;
wherein R⁹ is absent when Y² is N or is selected from H, halogen, C₁-C₆ alkyl and C₃-C₁₂ cycloalkyl when Y² is C; and
wherein R¹² is selected from NH₂, NHC₁-C₆ alkyl, C₁-C₆ alkyl, and C₁-C₆ heteroalkyl.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, C₁-C₆ alkyl, C₀-C₃ alkylOC₀-C₃ alkyl aryl wherein the aryl is optionally substituted by NH₂; C₀-C₃ alkylOC₀-C₃ alkyl heteroaryl wherein the heteroaryl is optionally substituted by NH₂;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁷ is absent when Y¹ is N or is H when Y¹ is C;
wherein R⁸ is absent when Y³ is N or is H when Y³ is C;
wherein R⁹ is absent when Y² is N or is H when Y² is C; and
wherein R¹² is NH₂.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H, C₁-C₆ alkyl, C₀-C₃ alkylOC₀-C₃ alkyl aryl wherein the aryl is optionally substituted by NH₂; C₀-C₃ alkylOC₀-C₃ alkyl heteroaryl wherein the heteroaryl is optionally substituted by NH₂;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁷ is absent when Y¹ is N or is H when Y¹ is C;
wherein R⁸ is absent when Y³ is N or is H when Y³ is C;
wherein R⁹ is absent when Y² is N or is H when Y² is C; and
wherein R¹² is NH₂.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl, C₃-C₁₂ cycloalkyl, aryl and heteroaryl wherein the aryl and the heteroaryl are optionally substituted by OC₁-C₆ alkyl, C₁-C₆ alkyl, C₁-C₆ heteroalkyl, halogen, C₁-C₆ alkyl C(O)R¹²;
wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂.

Further more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, halogen, C₁-C₆ alkyl, CN;
wherein R³ is selected from H, halogen and C₁-C₄ alkyl;
wherein R⁴, R⁵ and R⁶ are each independently selected from H, halogen, and C₁-C₄ alkyl; wherein R⁷ is absent when Y¹ is N or is selected from H, halogen and C₁-C₄ alkyl when Y¹ is C; wherein R⁸ is absent when Y³ is N or is selected from H, halogen and C₁-C₄ alkyl when Y³ is C; and
wherein R⁹ is absent when Y² is N or is selected from H, halogen and C₁-C₄ alkyl when Y² is C.

Further even more particular preferred compounds of formula (I) pharmaceutically-acceptable salts, hydrates, solvates, or stereoisomers thereof are those,
wherein X is selected from CH₂, CO, CHOH, CHO(C₁-C₃) alkyl, NH, and O, and is preferably O;
wherein Y¹ is N and R⁷ is absent, Y² is selected from N and C and Y³ is C;
wherein Z is NR¹⁰R¹¹, wherein R¹⁰ and R¹¹ are each independently selected from H and C₁-C₆ alkyl;
wherein R¹ is selected from H and C₁-C₆ alkyl;
wherein R² is selected from C₁-C₆ alkyl and C₃-C₁₂ cycloalkyl, and is preferably C₁-C₆ alkyl; wherein R³ is H;
wherein R⁴ is selected from H and halogen;
wherein R⁵ and R⁶ are each independently selected from H and C₁-C₆ alkyl;
wherein R⁸ is H;
wherein R⁹ is absent when Y² is N or is H when Y² is C; and
wherein R¹² is NH₂.

Even more particular preferred compounds of the compound of formula (I) are selected from the group consisting of

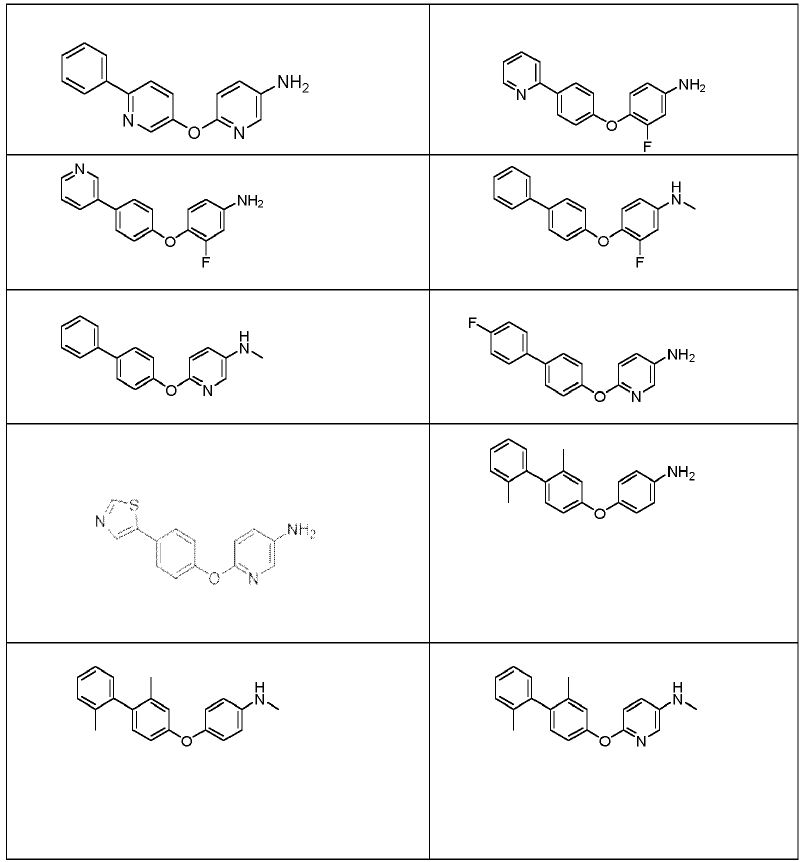

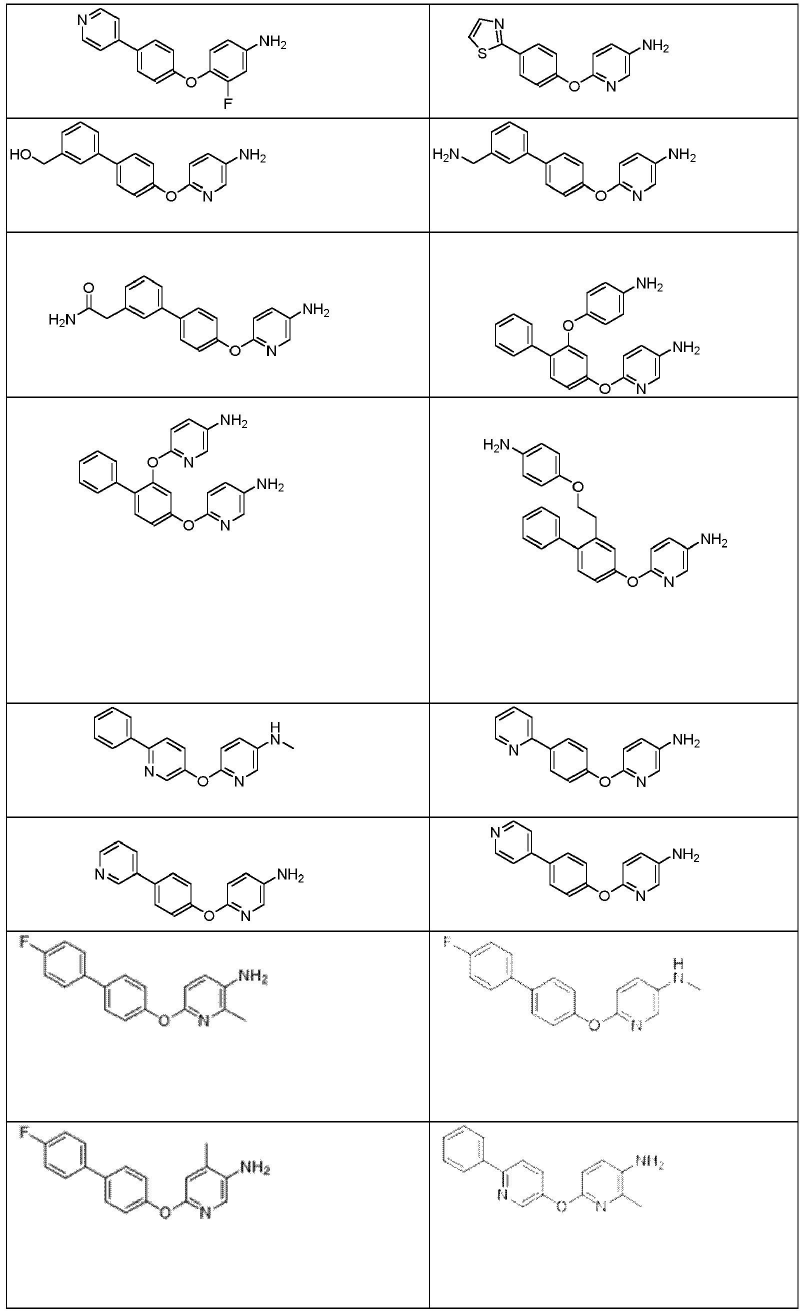

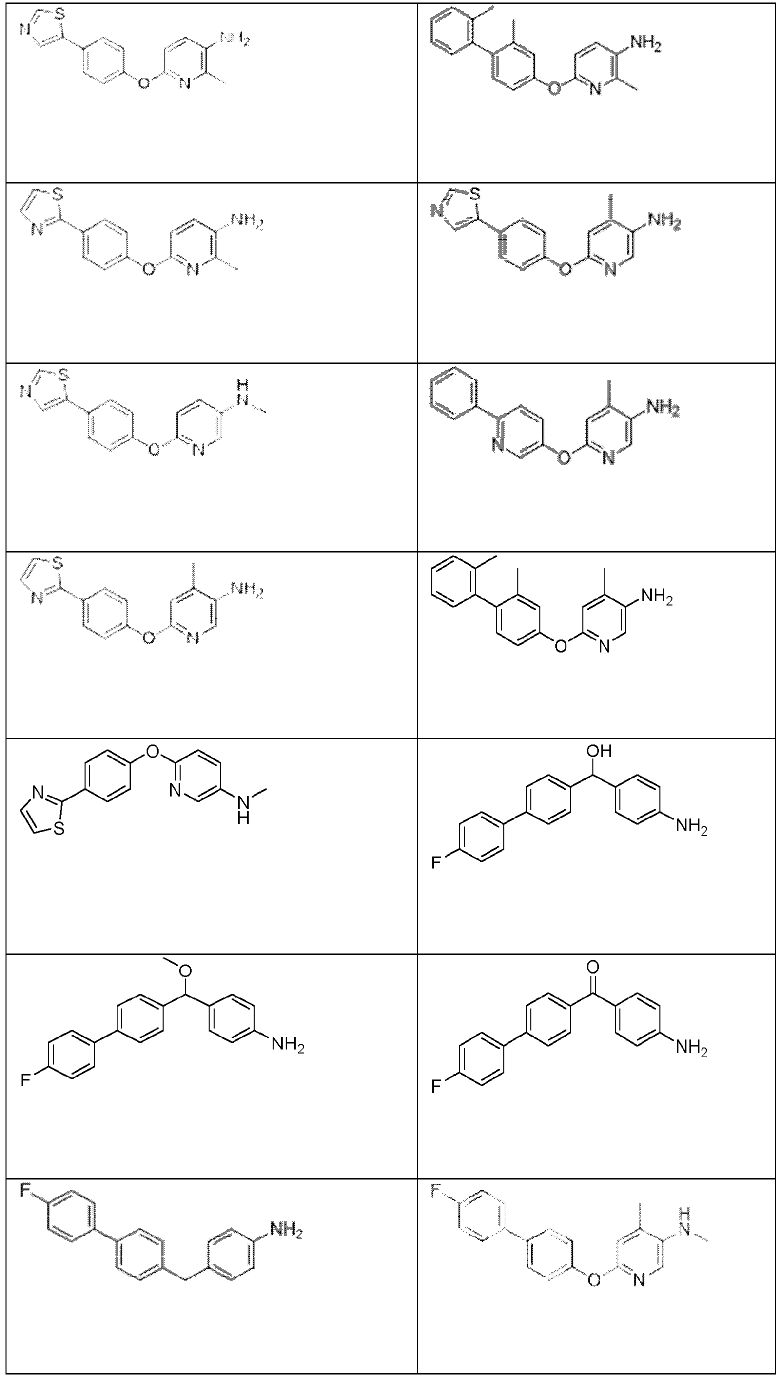

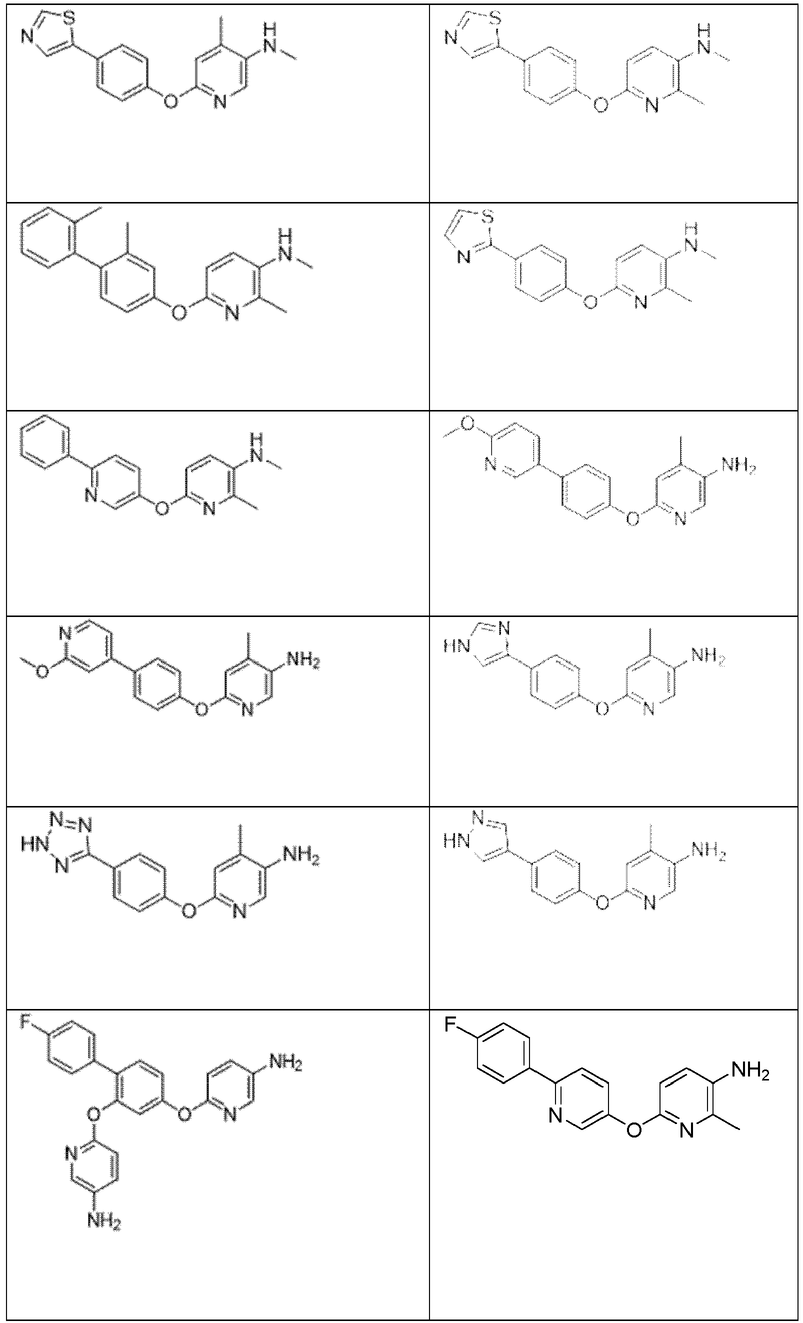

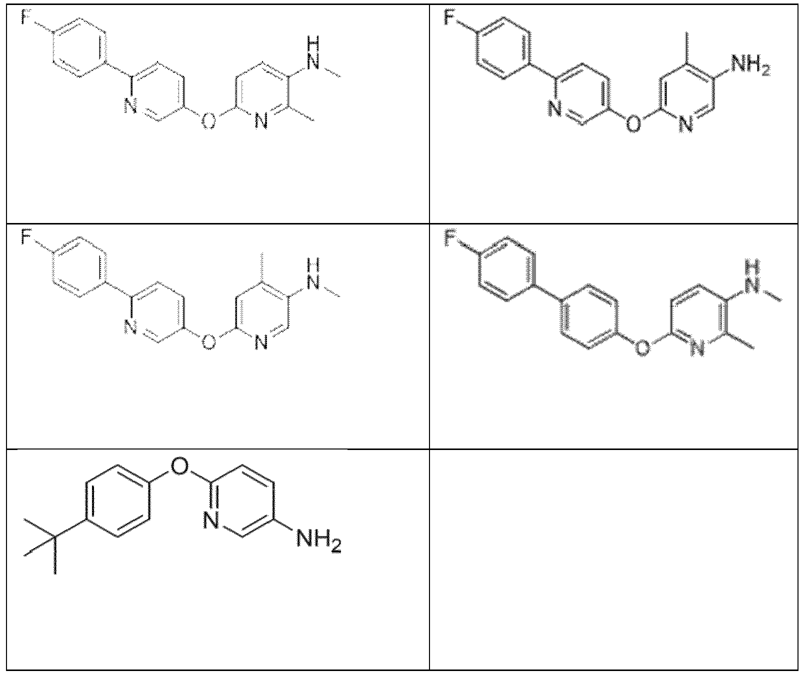

Even more particular preferred compounds of the compound of formula (I) are selected from the group consisting of

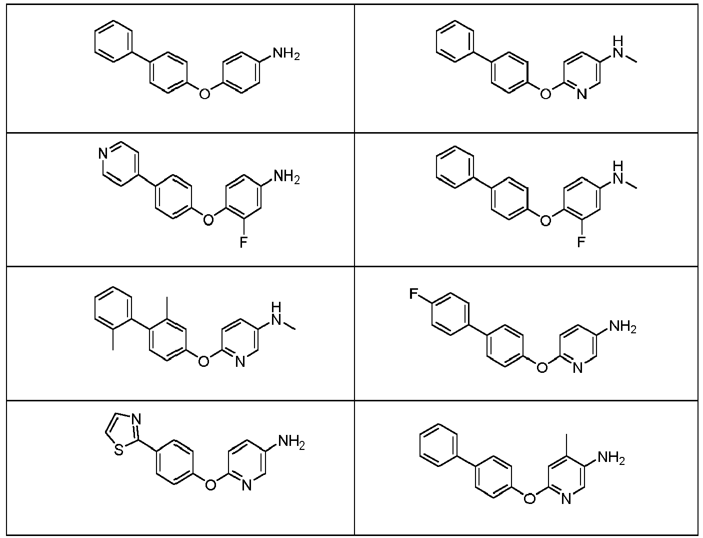

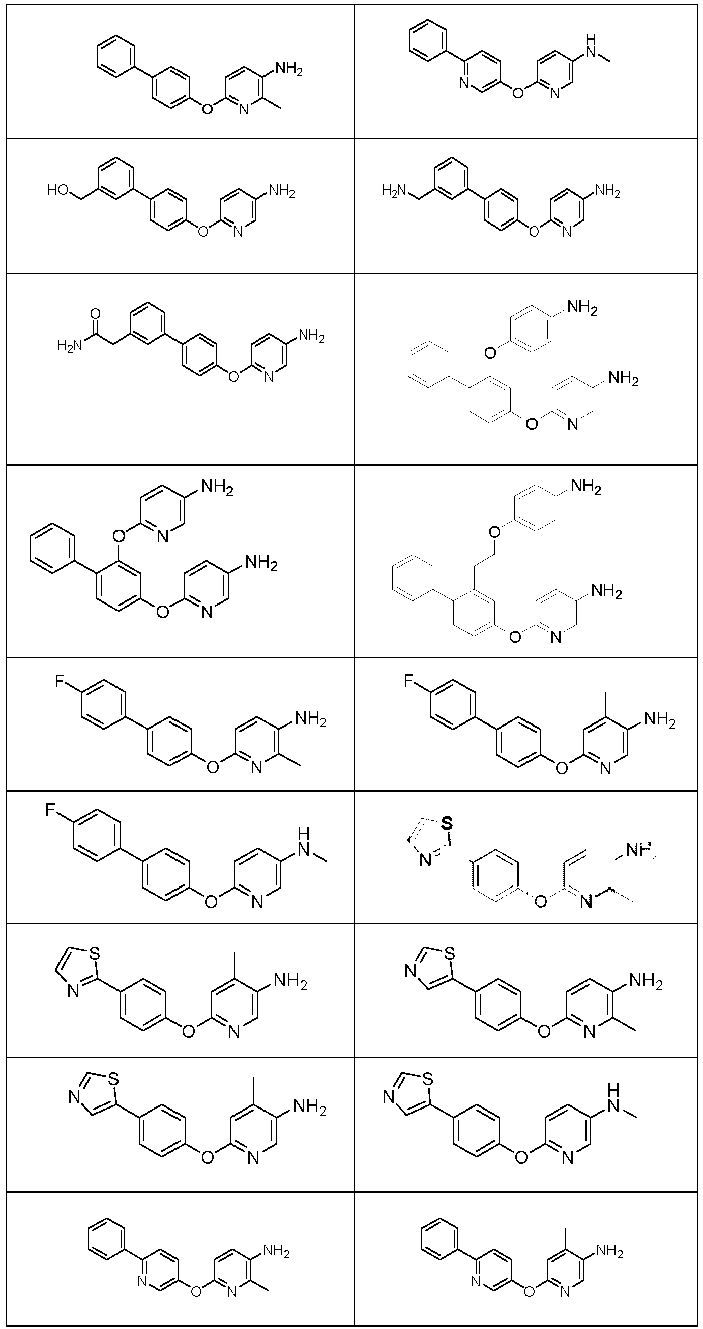

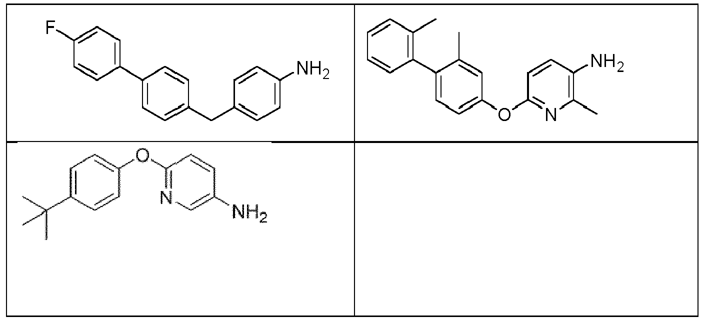

Most particular preferred compounds of the compound of formula (I) are selected from the group consisting of

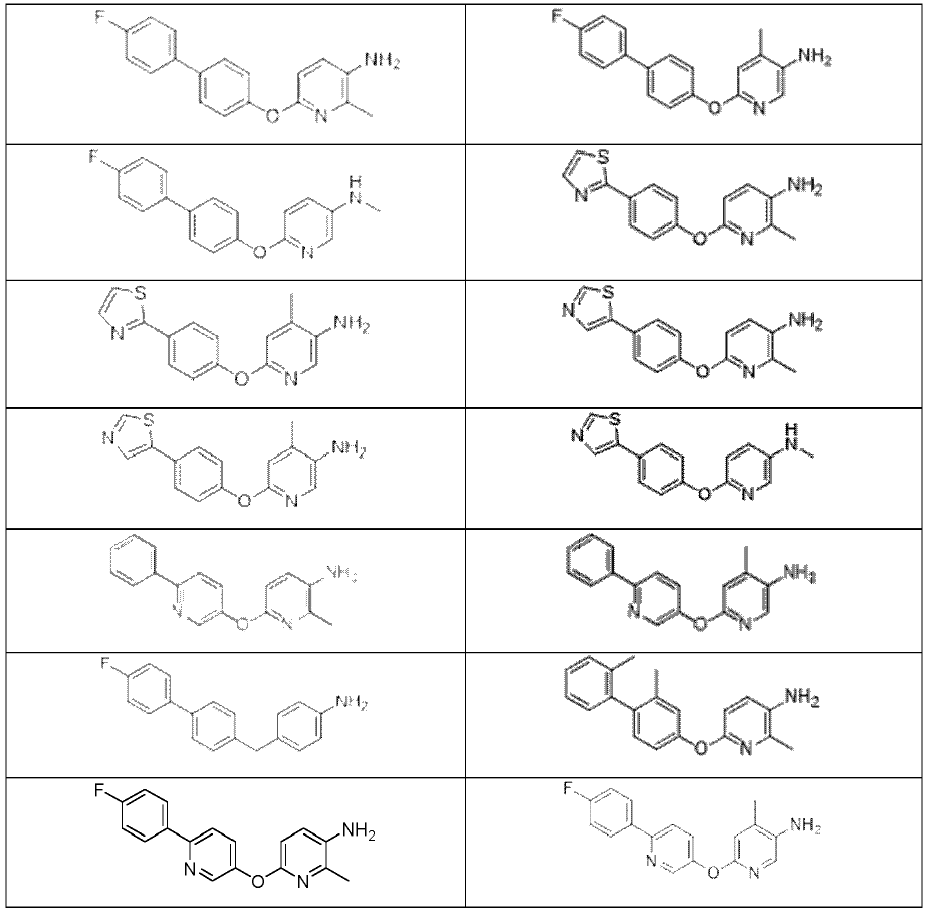

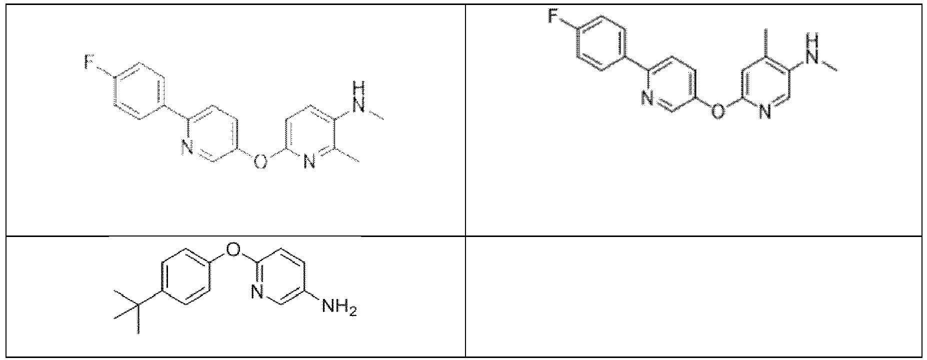

The most preferred NOTCH signaling pathway inhibitor is 6-(4-(tert-butyl)phenoxy)pyridin-3-amine or a pharmaceutically-acceptable salts, hydrates, solvates, or stereoisomers thereof.

In a further embodiment, there is provided a pharmaceutical composition according to the invention, comprising:
(a) a B-cell lymphoma 2 (BCL-2) inhibitor or a Myeloid cell leukemia-1 (MCL-1) inhibitor;
(b) a NOTCH signaling pathway inhibitor; and optionally
(c) one or more pharmaceutically acceptable diluents, excipients or carriers.

In a further embodiment, there is provided a pharmaceutical composition according to the invention, comprising:
(a) a BCL-2 inhibitor selected from the group consisiting of venetoclax, navitoclax, obatoclax, sabutoclax, ABT-737, PNT-2258, and S-055746, preferably venetoclax or navitoclax, more preferably navitoclax;
(b) a NOTCH signaling pathway inhibitor; and optionally
(c) one or more pharmaceutically acceptable diluents, excipients or carriers.

In a further embodiment, there is provided a pharmaceutical composition according to the invention, comprising:
(a) MCL-1 inhibitor selected from the group consisiting of S63845, AMG-176, AMG-397, and AZD5991, preferably S63845;
(b) a NOTCH signaling pathway inhibitor; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein selected from the group consisiting of venetoclax, navitoclax, obatoclax, sabutoclax, ABT-737, PNT-2258, S-055746, S63845, AMG-176, AMG-397, and AZD5991, preferably venetoclax, navitoclax or S63845, more preferably navitoclax or S63845;
(b) a NOTCH signaling pathway inhibitor; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein;
(b) a NOTCH signaling pathway inhibitor selected from the group consisting of a compound of formula (I), a γ-secretase inhibitor, a blocking antibody against NOTCH receptors, a blocking antibody against NOTCH ligands, and an inhibitor of NOTCH transcription complex; and optionally
(c) one or more pharmaceutically acceptable diluents, excipients or carriers.

In a further embodiment, there is provided a pharmaceutical composition according to the invention, comprising:
(a) a B-cell lymphoma 2 (BCL-2) inhibitor or a Myeloid cell leukemia-1 (MCL-1) inhibitor;
(b) a NOTCH signaling pathway inhibitor selected from the group consisting of a compound of formula (I), a γ-secretase inhibitor, a blocking antibody against NOTCH receptors, a blocking antibody against NOTCH ligands, and an inhibitor of NOTCH transcription complex; and optionally
(c) one or more pharmaceutically acceptable diluents, excipients or carriers.

In a further embodiment, there is provided a pharmaceutical composition according to the invention, comprising:
(a) a BCL-2 inhibitor selected from the group consisiting of venetoclax, navitoclax, obatoclax, sabutoclax, ABT-737, PNT-2258, and S-055746, preferably venetoclax or navitoclax, more preferably navitoclax;
(b) a NOTCH signaling pathway inhibitor selected from the group consisting of a compound of formula (I), a γ-secretase inhibitor, a blocking antibody against NOTCH receptors, a blocking antibody against NOTCH ligands, and an inhibitor of NOTCH transcription complex; and optionally
(c) one or more pharmaceutically acceptable diluents, excipients or carriers.

In a further embodiment, there is provided a pharmaceutical composition according to the invention, comprising:
(a) MCL-1 inhibitor selected from the group consisiting of S63845, AMG-176, AMG-397, and AZD5991, preferably S63845;
(b) a NOTCH signaling pathway inhibitor selected from the group consisting of a compound of formula (I), a γ-secretase inhibitor, a blocking antibody against NOTCH receptors, a blocking antibody against NOTCH ligands, and an inhibitor of NOTCH transcription complex; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein selected from the group consisiting of venetoclax, navitoclax, obatoclax, sabutoclax, ABT-737, PNT-2258, S-055746, S63845, AMG-176, AMG-397, and AZD5991, preferably venetoclax, navitoclax or S63845, more preferably navitoclax or S63845;
(b) a NOTCH signaling pathway inhibitor selected from the group consisting of a compound of formula (I), a γ-secretase inhibitor, a blocking antibody against NOTCH receptors, a blocking antibody against NOTCH ligands, and an inhibitor of NOTCH transcription complex; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein;
(b) a NOTCH signaling pathway inhibitor selected from the group consisting of a γ-secretase inhibitor and a compound of formula (I); and optionally
(c) one or more pharmaceutically acceptable diluents, excipients or carriers.

In a further embodiment, there is provided a pharmaceutical composition according to the invention, comprising:
(a) a B-cell lymphoma 2 (BCL-2BCL-2) inhibitor or a Myeloid cell leukemia-1 (MCL-1) inhibitor;
(b) a NOTCH signaling pathway inhibitor selected from the group consisting of a γ-secretase inhibitor and a compound of formula (I); and optionally
(c) one or more pharmaceutically acceptable diluents, excipients or carriers.

In a further embodiment, there is provided a pharmaceutical composition according to the invention, comprising:
(a) a BCL-2BCL-2 inhibitor selected from the group consisiting of venetoclax, navitoclax, obatoclax, sabutoclax, ABT-737, PNT-2258, and S-055746, preferably venetoclax or navitoclax, more preferably navitoclax;
(b) a NOTCH signaling pathway inhibitor selected from the group consisting of a γ-secretase inhibitor and a compound of formula (I); and optionally
(c) one or more pharmaceutically acceptable diluents, excipients or carriers.

In a further embodiment, there is provided a pharmaceutical composition according to the invention, comprising:
(a) MCL-1 inhibitor selected from the group consisiting of S63845, AMG-176, AMG-397, and AZD5991, preferably S63845;
(b) a NOTCH signaling pathway inhibitor selected from the group consisting of a γ-secretase inhibitor and a compound of formula (I); and optionally
(c) one or more pharmaceutically acceptable diluents, excipients or carriers.

In a further embodiment, there is provided a pharmaceutical composition according to the invention, comprising:
(a) an inhibitor of an anti-apoptotic protein selected from the group consisiting of venetoclax, navitoclax, obatoclax, sabutoclax, ABT-737, PNT-2258, S-055746, S63845, AMG-176, AMG-397, and AZD5991, preferably venetoclax, navitoclax or S63845, more preferably navitoclax or S63845;
(b) a NOTCH signaling pathway inhibitor selected from the group consisting of a γ-secretase inhibitor and a compound of formula (I); and optionally
(c) one or more pharmaceutically acceptable diluents, excipients or carriers.

In a further embodiment, there is provided a pharmaceutical composition according to the invention, comprising:
(a) an inhibitor of an anti-apoptotic protein;
(b) a compound of formula I as defined herein or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) a B-cell lymphoma 2 (BCL-2BCL-2) inhibitor or a Myeloid cell leukemia-1 (MCL-1) inhibitor;
(b) a compound of formula I as defined herein or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) a BCL-2BCL-2 inhibitor selected from the group consisiting of venetoclax, navitoclax, obatoclax, sabutoclax, ABT-737, PNT-2258, and S-055746, preferably venetoclax or navitoclax, more preferably navitoclax;
(b) a compound of formula I as defined herein or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) MCL-1 inhibitor selected from the group consisiting of S63845, AMG-176, AMG-397, and AZD5991, preferably S63845;
(b) a compound of formula I as defined herein or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein selected from the group consisiting of venetoclax, navitoclax, obatoclax, sabutoclax, ABT-737, PNT-2258, S-055746, S63845, AMG-176, AMG-397, and AZD5991, preferably venetoclax, navitoclax or S63845, more preferably navitoclax or S63845;
(b) a compound of formula I as defined herein or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein;
(b) 6-(4-(tert-butyl)phenoxy)pyridin-3-amine or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) a B-cell lymphoma 2 (BCL-2) inhibitor or a Myeloid cell leukemia-1 (MCL-1) inhibitor;
(b) 6-(4-(tert-butyl)phenoxy)pyridin-3-amine or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) a BCL-2 inhibitor selected from the group consisiting of venetoclax, navitoclax, obatoclax, sabutoclax, ABT-737, PNT-2258, and S-055746, preferably venetoclax or navitoclax, more preferably navitoclax;
(b) 6-(4-(tert-butyl)phenoxy)pyridin-3-amine or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) MCL-1 inhibitor selected from the group consisiting of S63845, AMG-176, AMG-397, and AZD5991, preferably S63845;
(b) 6-(4-(tert-butyl)phenoxy)pyridin-3-amine or pharmaceutically-acceptable salts, hydrates, solvates, or stereoisomers thereof; and optionally
(c) one or more pharmaceutically acceptable diluents, excipients or carriers.

In a further embodiment, there is provided a pharmaceutical composition according to the invention, comprising:
(a) an inhibitor of an anti-apoptotic protein selected from the group consisiting of venetoclax, navitoclax, obatoclax, sabutoclax, ABT-737, PNT-2258, S-055746, S63845, AMG-176, AMG-397, and AZD5991, preferably venetoclax, navitoclax or S63845, more preferably navitoclax or S63845;
(b) 6-(4-(tert-butyl)phenoxy)pyridin-3-amine or pharmaceutically-acceptable salts, hydrates, solvates, or stereoisomers thereof; and optionally
(c) one or more pharmaceutically acceptable diluents, excipients or carriers.

### Combinations:

As outlined above, the invention relates to a pharmaceutical combination comprising an inhibitor of an anti-apoptotic protein, such as a BCL-2 inhibitor or a MCL-1 inhibitor and a NOTCH signaling pathway inhibitor, such as 6-(4-(tert-butyl)phenoxy)pyridin-3-amine or a pharmaceutically-acceptable salts, hydrates, solvates, or stereoisomers thereof. A pharmaceutical combination according to the invention is for example a combined preparation or a pharmaceutical composition, for simultaneous, separate or sequential use.

The term "combined preparation" as used herein defines especially a "kit of parts" in the sense that said inhibitor of an anti-apoptotic protein and said NOTCH signaling pathway inhibitor can be dosed independently, either in separate form or by use of different fixed combinations with distinguished amounts of the active ingredients. The ratio of the amount of inhibitor of an anti-apoptotic protein to the amount of NOTCH signaling pathway inhibitor to be administered in the combined preparation can be varied, e.g. in order to cope with the needs of a patient subpopulation to be treated or the needs of a single patient, which needs can be different due to age, sex, body weight, etc. of a patient. The individual parts of the combined preparation (kit of parts) can be administered simultaneously or sequentially, i.e. chronologically staggered, e.g. at different time points and with equal or different time intervals for any part of the kit of parts.

The term "pharmaceutical composition" refers to a fixed-dose combination (FDC) that includes the inhibitor of an anti-apoptotic protein and the NOTCH signaling pathway inhibitor combined in a single dosage form, having a predetermined combination of respective dosages.

The pharmaceutical combination further may be used as add-on therapy. As used herein, "add-on" or "add-on therapy" means an assemblage of reagents for use in therapy, the subject receiving the therapy begins a first treatment regimen of one or more reagents prior to beginning a second treatment regimen of one or more different reagents in addition to the first treatment regimen, so that not all of the reagents used in the therapy are started at the same time. For example, adding NOTCH signaling pathway inhibitor therapy to a patient already receiving inhibitor of an anti-apoptotic protein therapy.

In a preferred embodiment, the pharmaceutical combination according to the invention is a pharmaceutical composition, i.e. a fixed-dose combination.

In a further preferred embodiment, the pharmaceutical combination according to the invention is a combined preparation.

The amount of the inhibitor of an anti-apoptotic protein and the NOTCH signaling pathway inhibitor to be administered will vary depending upon factors such as the particular compound, disease condition and its severity, according to the particular circumstances surrounding the case, including, e.g., the specific inhibitor of an anti-apoptotic protein being administered, the route of administration, the condition being treated, the target area being treated, and the subject or host being treated.

In one embodiment, the invention provides a pharmaceutical combination comprising an inhibitor of an anti-apoptotic protein and a NOTCH signaling pathway inhibitor, wherein said inhibitor of an anti-apoptotic protein and said NOTCH signaling pathway inhibitor are present in a therapeutically effective amount.

The expression "effective amount" or "therapeutically effective amount" as used herein refers to an amount capable of invoking one or more of the following effects in a subject receiving the combination of the present invention: (i) inhibition or arrest of tumor growth, including, reducing the rate of tumor growth or causing complete growth arrest; (ii) reduction in the number of tumor cells; (iii) reduction in tumor size; (iv) reduction in tumor number; (v) inhibition of metastasis (i.e., reduction, slowing down or complete stopping) of tumor cell infiltration into peripheral organs; (vi) enhancement of antitumor immune response, which may, but does not have to, result in the regression or elimination of the tumor; (vii) relief, to some extent, of one or more symptoms associated with cancer; (viii) increase in progression-free survival (PFS) and/or; overall survival (OS) of the subject receiving the combination.

Determination of a therapeutically effective amount is well within the capability of those skilled in the art, especially in light of the detailed disclosure provided herein. In some embodiments, a therapeutically effective amount may (i) reduce the number of cancer cells; (ii) reduce tumor size; (iii) inhibit, retard, slow to some extent, and preferably stop cancer cell infiltration into peripheral organs; (iv) inhibit (e.g., slow to some extent and preferably stop) tumor metastasis; (v) inhibit tumor growth; (vi) delay occurrence and/or recurrence of a tumor; and/or (vii) relieve to some extent one or more of the symptoms associated with the cancer. In various embodiments, the amount is sufficient to ameliorate, palliate, lessen, and/or delay one or more of symptoms of cancer.

In another preferred embodiment, the invention provides a pharmaceutical combination comprising an inhibitor of an anti-apoptotic protein and a NOTCH signaling pathway inhibitor, wherein said inhibitor of an anti-apoptotic protein and said NOTCH signaling pathway inhibitor are present in an amount producing an additive therapeutic effect.

As used herein, the term "additive" means that the effect achieved with the pharmaceutical combinations of this invention is approximately the sum of the effects that result from using the anti-cancer agents, namely the inhibitor of an anti-apoptotic protein and the NOTCH signaling pathway inhibitor, as a monotherapy. Advantageously, an additive effect provides for greater efficacy at the same doses, and may lead to longer duration of response to the therapy.

In another preferred embodiment, the invention provides a pharmaceutical combination comprising an inhibitor of an anti-apoptotic protein and a NOTCH signaling pathway inhibitor, wherein said inhibitor of an anti-apoptotic protein and said NOTCH signaling pathway inhibitor are present in an amount producing a synergistic therapeutic effect.

As used herein, the term "synergistic" means that the effect achieved with the pharmaceutical combinations of this invention is greater than the sum of the effects that result from using the anti-cancer agents, namely the inhibitor of an anti-apoptotic protein and the NOTCH signaling pathway inhibitor, as a monotherapy. Advantageously, such synergy provides for greater efficacy at the same doses, and may lead to longer duration of response to the therapy.

In one embodiment, the invention provides a pharmaceutical combination comprising a NOTCH signaling pathway inhibitor and an inhibitor of an anti-apoptotic protein, wherein the amount of said NOTCH signaling pathway inhibitor in the combination is from about 1 to about 1000 mg.

In a preferred embodiment, the invention provides a pharmaceutical combination comprising a NOTCH signaling pathway inhibitor and an inhibitor of an anti-apoptotic protein, wherein the amount of said inhibitor of an anti-apoptotic protein in the combination is from about 0.1 to about 100 mg.

### Formulations and modes of administration:

A pharmaceutical combination according to the invention is, preferably, suitable for enteral administration, such as oral or rectal administration to a subject and comprises a therapeutically effective amount of the active ingredients and one or more suitable pharmaceutically acceptable carrier.

If not indicated otherwise, a pharmaceutical combination according to the invention is prepared in a manner known per se, e.g. by means of conventional mixing, granulating, coating, dissolving or lyophilizing processes. In preparing a combination for an oral dosage form, any of the usual pharmaceutical media may be employed, for example water, glycols, oils, alcohols, carriers, such as starches, sugars, or microcrystalline cellulose, diluents, granulating agents, lubricants, binders, disintegrating agents and the like. Because of their ease of administration, tablets and capsules represent the most advantageous oral dosage unit forms, in which case solid pharmaceutical carriers are obviously employed.

In one embodiment, the pharmaceutical combination according to the invention is a combination for enteral administration. Preferred are combinations for oral administration. As indicated above, said pharmaceutical combination is preferably a pharmaceutical composition, i.e. fixed-dose combination.

A pharmaceutical combination for enteral administration is, for example, a unit dosage form, such as a tablet, a capsule or a suppository.

In one embodiment, the invention provides a pharmaceutical composition comprising an inhibitor of an anti-apoptotic protein, such as a BCL-2 inhibitor or a MCL-1 inhibitor and a NOTCH signaling pathway inhibitor, such as 6-(4-(tert-butyl)phenoxy)pyridin-3-amine and at least one pharmaceutically acceptable carrier, wherein the composition is a tablet or a capsule, preferably a tablet.

In a preferred embodiment, the invention provides a pharmaceutical composition comprising an inhibitor of an anti-apoptotic protein, such as a BCL-2 inhibitor or a MCL-1 inhibitor and a NOTCH signaling pathway inhibitor, such as 6-(4-(tert-butyl)phenoxy)pyridin-3-amine and at least one pharmaceutically acceptable carrier, wherein the composition is a sustained release tablet.

In a further preferred embodiment, the pharmaceutical composition according to the invention is for oral administration, wherein the composition is adapted to provide sustained release of the active pharmaceutical ingredients (API). Thus, the composition may increase Tₘₐₓ or reduce Cₘₐₓ, or both increase Tₘₐₓ and reduce Cₘₐₓ, as compared to an immediate release composition.

"Cₘₐₓ" means the the peak concentration of the drug in the plasma. "Tₘₐₓ" means the time from administration to reach Cₘₐₓ.

A sustained release composition as compared to an immediate release composition comprises one or more agents which act to prolong release of the API; for example, the API may be embedded in a matrix and/or surrounded by a membrane which, in either case, controls (reduces) the rate of diffusion of the API into the Gl tract.

Additional or alternative, e.g. alternative materials which may be included in the composition to provide sustained release are hydrophobic polymers, for example ethyl cellulose or a methacrylic acid polymer, or a combination thereof. Such polymers, whether used singly or in combination, may be comprised in a coating or may be included in admixture with the API (i.e. may be used as a matrix-former), or may be present both in a coating and in admixture with the API.

Further additional or alternative, e.g. alternative materials which may be included in the composition to provide sustained release are insoluble erodible materials, for example a wax or a hydrogenated vegetable oil, or a combination thereof. Such materials, whether used singly or in combination, may be comprised in a coating or may be included in admixture with the API (i.e. may be used as a matrix-former), or may be present both in a coating and in admixture with the API.

The unit content of active ingredients in an individual dose need not in itself constitute a therapeutically effective amount, since such an amount can be reached by the administration of a plurality of dosage units. A composition according to the invention may contain, e.g., from about 10% to about 100% of the therapeutically effective amount of the active ingredients.

Where the pharmaceutical combination according to the invention is a combined preparation, said inhibitor of an anti-apoptotic protein need not be administered in the same dosage form as said NOTCH signaling pathway inhibitor.

### Dosing regimen:

An exemplary treatment regime entails administration once daily, twice daily, three times daily, every second day, twice per week, once per week. The combination of the invention is usually administered on multiple occasions. Intervals between single dosages can be, for example, less than a day, daily, every second day, twice per week, or weekly. The combination of the invention may be given as a continous uninterrupted treatment. The combination of the invention may also be given in a regime in which the subject receives cycles of treatment interrupted by a drug holiday or period of non-treatment. Thus, the combination of the invention may be administered according to the selected intervals above for a continuous period of one week or a part thereof, for two weeks, for three weeks, for four weeks, for five weeks or for six weeks and then stopped for a period of one week, or a part thereof, for two weeks, for three weeks, for four weeks, for five weeks, or for six weeks. The combination of the treatment interval and the non-treatment interval is called a cycle. The cycle may be repeated one or more times. Two or more different cycles may be used in combination for repeating the treatment one or more times. Intervals can also be irregular as indicated by measuring blood levels of said inhibitor of an anti-apoptotic protein and/or said NOTCH signaling pathway inhibitor in the patient. In a preferred embodiment, the pharmaceutical combination according to the invention is administered once daily. In an exemplary treatment regime the inhibitor of an anti-apoptotic protein can be administered from 0.1 - 100 mg per day and the NOTCH signaling pathway inhibitor can be administered from 1 - 1000 mg per day.

### Using the combinations of the invention to treat cancer

According to a second aspect the present invention provides a pharmaceutical combination as described herein, for use as a medicament.

According to a third aspect the present invention provides a pharmaceutical combination as described herein, for use in a method for the prevention, delay of progression or treatment of cancer in a subject.

Also provided is the use of a pharmaceutical combination as described herein for the manufacture of a medicament for the prevention, delay of progression or treatment of cancer in a subject.

Also provided is the use of a pharmaceutical combination as described herein for the prevention, delay of progression or treatment of cancer in a subject.

Also provided is a method for the prevention, delay of progression or treatment of cancer in a subject, comprising administering to said subject a therapeutically effective amount of a pharmaceutical combination as described herein.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

As used herein, "delay of progression" means increasing the time to appearance of a symptom of a cancer or a mark associated with a cancer or slowing the increase in severity of a symptom of a cancer. Further, "delay of progression" as used herein includes reversing or inhibition of disease progression. "Inhibition" of disease progression or disease complication in a subject means preventing or reducing the disease progression and/or disease complication in the subj ect.

Preventive treatments comprise prophylactic treatments. In preventive applications, the pharmaceutical combination of the invention is administered to a subject suspected of having, or at risk for developing cancer. In therapeutic applications, the pharmaceutical combination is administered to a subject such as a patient already suffering from cancer, in an amount sufficient to cure or at least partially arrest the symptoms of the disease. Amounts effective for this use will depend on the severity and course of the disease, previous therapy, the subject's health status and response to the drugs, and the judgment of the treating physician.
In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.
In the case wherein the subject's status does improve, the pharmaceutical combination may be administered continuously; alternatively, the dose of drugs being administered may be temporarily reduced or temporarily suspended for a certain length of time (i.e., a "drug holiday").
Once improvement of the patient's condition has occurred, a maintenance dose of the pharmaceutical combination of the invention is administered if necessary. Subsequently, the dosage or the frequency of administration, or both, is optionally reduced, as a function of the symptoms, to a level at which the improved disease is retained.
In a preferred embodiment the cancer is a NOTCH-dependent cancer.

The term "NOTCH-dependent" or "NOTCH-dependent cancer" as used herein refers to a NOTCH signaling pathway activated cancer. The NOTCH signaling pathway is one of the most commonly activated signaling pathways in cancer. A "NOTCH-dependent cancer" can have one or more of the following genetic causes:
- Chromosomal translocation overlapping with NOTCH regions on the chromosomes, leading to fusion of NOTCH related genes and expression of truncated versions of the NOTCH 1,2, 3 and/or 4 proteins, causing ligand-/receptor-independent constitutive activation_of the NOTCH pathway.
- Gain of function (GOF) mutations in the *NOTCH* genes leading to a constitutive, ligand-/receptor-independent activation of the NOTCH pathway.
- Loss of function (LOF) mutations in negative regulators of the NOTCH pathway, like FBXW7 or NUMB.
- Over-expression of the different NOTCH-specific ligands or receptors, leading to extended activation of the NOTCH pathway.

More preferably according to the present invention, cancers are NOTCH-dependent cancers that can be either solid tumours or hematological malignancies. In one embodiment, the NOTCH-dependent cancer is resistant to γ-secretase inhibitor treatment. Examples of γ-secretase inhibitor treatment comprise 1) Gamma secretase inhibitor RO4929097 and Cediranib Maleate in treating patients with advanced solid tumours (NCT01131234), 2) Gamma-Secretase Inhibitor RO4929097 in Treating Young Patients With Relapsed or Refractory Solid Tumours, CNS Tumours, Lymphoma, or T-Cell Leukemia (NCT01088763), 3) Study of MK-0752 in combination with Tamoxifen or Letrozole to treat early stage breast cancer (NCT00756717), 4) GDC-0449 and RO4929097 in treating patients with Advances or metastatic sarcoma (NCT01154452) 5) RO4929097 and Erlotinib Hydrochloride in treating patients with stage IV or recurrent Non-Small Cell Lung Cancer (NCT01193881), 6) Bicalutamide and RO4929097 in treating patients with previously treated prostate cancer (NCT01200810), 7) RO4929097 in treating patients with recurrent invasive Gliomas (NCT01269411), 8) A NOTCH signaling pathway inhibitor for patients with T-cell Acute Lymphoblastic Leukemia/Lymphoma (ALL) (NCT00100152) and 9) RO4929097 in treating patients with metastatic colorectal cancer (NCT01116687).

In a more preferred embodiment the cancer is a solid tumor. Preferably the solid tumor is selected from the group consisting of adenoid cystic carcinoma (ACC), breast cancer, prostate cancer, osteosarcoma, malignant glomus tumour, colorectal cancer and hepatocellular carcinoma.

Breast cancer is usually selected from the group consisting of Estrogen receptor positive (ER+), Estrogen receptor negative (ER-), Progesteron receptor positive (PR+), Progesteron receptor negative (PR-), human epidermal growth factor receptor 2 positive (HER2+), human epidermal growth factor receptor 2 negative (HER2-) breast cancers, and triple negative breast cancer (TNBC) and is preferably triple negative breast cancer (TNBC).

In a further more preferred embodiment the cancer is a haematological cancer. Preferably the haematological cancer is selected from the group consisting of acute lymphoblastic leukemia, T-cell acute lymphoblastic leukemia or T-cell lymphoblastic lymphoma, acute myeloid leukemia, chronic lymphocytic leukemia, chronic myelocytic leukemia, Hodgkin lymphoma, non-Hodgkin lymphoma, follicular lymphoma, diffuse large B cell lymphoma, Burkitt lymphoma, marginal zone B-cell lymphoma, splenic marginal zone lymphoma, mantle cell lymphoma, peripheral T-cell lymphoma, anaplastic large cell lymphoma, CNS lymphoma, or multiple myeloma

In a particular preferred embodiment the cancer is selected from the group consisting of T-cell acute lymphoblastic leukemia, breast cancer and adenoid cystic carcinoma (ACC), and is preferably T-cell acute lymphoblastic leukemia and breast cancer, more preferably T-cell acute lymphoblastic leukemia and triple negative breast cancer (TNBC).

### Examples

The present examples are intended to illustrate the present invention without restricting it.

### EXAMPLE 1

### In vitro assay of combinations of a BCL-2 inhibitor or a MCL-1 inhibitor with a NOTCH signaling pathway inhibitor

**Cell lines and drug combinations:** Human cancer cell lines RPMI-8402, HCC1187 and MCF-7 were cultured under mycoplasma free conditions at 37°C in RPMI-1640 media supplemented with 10% FCS (RPMI-8402 and HCC1187 cells) and DMEM media supplemented with 10% FCS (MCF-7 cells). To determine effect of 6-(4-(tert-butyl)phenoxy)pyridin-3-amine with inhibitors of anti-apoptotic proteins, drugs listed in table 1 were used.

**Table 1: inhibitors of anti-apoptotic proteins used in combination with 6-(4-(tert-butyl)phenoxy)pyridin-3 -amine.**

| **Drug** | **Target** / **Mode of Action** |
|---|---|
| ABT-263 (Navitoclax) | BCL-2 inhibitor (BCL-2i) |
| S63845 | MCL-1 inhibitor (MCL-1i) |

**Determination of optimal positive control for screening:** In order to identify an appropriate positive control to induce significant cell death and retard cell proliferation, 3 cancer cell lines were treated with 2 different compounds i.e gambogic acid and bortezomib. The sensitivity and reproducibility of the assay was determined by calculating Z' prime values. Based on a Z' value between 0.5 and 1.0, gambogic acid was selected as a positive control for MCF-7 cells and bortezomib was used as a positive control for HCC1187 and RPMI-8402 cells.
**Single agent screening:** Human cancer cell lines HCC1187, MCF-7 and RPMI-8402 were seeded at a density of 1500 cells/well in 384 well plate format and cultured in 40 µl of growth media. Cells were treated with each of the listed compounds in table 1 as single agent with a concentration ranging from 10 nM to 10 µM in duplicates. Following treatment for 72 hours, cells were incubated with alamarBlue^{®} for 4 hours and cell proliferation readout was taken using Infinite^{®} F500 (Tecan) plate reader. All volumes (cells and alamarBlue^{®}) were dispensed using Multidrop Combi dispenser using a standard cassette (Speed MEDIUM).
**6-(4-(tert-butyl)phenoxy)pyridin-3-amine combination screening:** Human cancer cell lines HCC1187, MCF-7 and RPMI-8402 were seeded at a density of 1500 cells/well in 384 well plate format and cultured in 40 µl of growth media. Cells were treated with a combination of 6-(4-(tert-butyl)phenoxy)pyridin-3-amine (concentration range from 150 nM to 10 µM) and chemical compounds listed in table 1 (concentration range from 75 nM to 20 µM) for 72 hours. Concentration range used for each compound was determined based on individual IC₅₀ values i.e concentration range selected flanks IC₅₀ value of each compound. Each of the 3 compounds were combined with 6-(4-(tert-butyl)phenoxy)pyridin-3-amine at different concentrations generating an 8X10 matrix in duplicates. alamarBlue^{®} readout was taken using Infinite^{®} F500 (Tecan) plate reader. All volumes (cells and alamarBlue^{®}) were dispensed using Multidrop Combi dispenser using a standard cassette (Speed MEDIUM).
**Compound management and dispensing:** All compounds were prepared as stock solution at 10 mM in pure DMSO. Purity was checked by LC/MS and was above 90% for all solutions. To create 384 well working plates, different volumes of stock solutions were plated into 384 well plates by using ECHO 550 acoustic dispenser (Labcyte) to generate the final concentration of interest for each drug, either alone or in matrix combination.
**AlamarBlue^{®} viability assay:** alamarBlue^{®} proliferation assays were performed to determine the growth kinetics of compound treated cells. alamarBlue^{®} consists of a cell permeable substrate resazurin. In metabolically active and proliferating cells, resazurin is converted to resorufin due to an intrinsic reducing power of live cells and produces a red fluorescence. Therefore, production of resorufin serves as an indicator of the viability of the cell population. Proliferation assays were performed by seeding 1500 cells/well in 384 well plate format. Cells were treated in duplicates with pre-determined concentration of compounds as a single agent or in combination with 6-(4-(tert-butyl)phenoxy)pyridin-3-amine for 72 hours. To determine growth kinetics, alamarBlue^{®} (Invitrogen) was added to each well and incubated for 4 hours. alamarBlue^{®} readout was taken using Infinite^{®} F500 (Tecan) plate reader.
**Data analysis for individual compounds:** To determine anti-proliferative activity and IC₅₀ values of individual compounds (listed in table 1) against HCC1187, MCF-7 and RPMI-8402 cells, data were analyzed using GraphPad Prism.
**Data analysis for combination screening:** Synergistic interaction analysis was performed using SynergyFinder software (http://bioconductor.org/packages/release/bioc/html/synergyfinder.html or https://synergyfinder.fimm.fi/) as described by Ianevsky et al (2017)¹. Analysis was performed according to the Highest single agent (HSA) model, which states that the expected effect from a combination of compounds equals to the higher effect of individual drugs at the dose in the combination (Gaddum, *Pharmacology,* 1940). Hence any additional effect over the higher single drug is considered as synergy. To mitigate the impact of edge-effects, correction was applied for all analyses. Regions of interest were defined as concentration combination showing both synergistic interaction and significant absolute activity (> 60%).

### Results and conclusions

Determination of IC₅₀ values for each of the drug in 3 cancer cell lines: IC₅₀ values were used as a measure of anti-proliferative activity of investigated compounds. The data is summarized in Table 2. The IC₅₀ values for each compound vary from cell line to cell line reflecting genomic profile of each cell line and target specificity of each compound.

**Table 2: IC₅₀ (µM) values of drugs used as single agent in three human cancer cell lines.**

| | RPMI-8402 | HCC1187 | MCF-7 |
|---|---|---|---|
| ABT-263 (Navitoclax) | 5.5 | 20 | 21 |
| S63845 | 0.04 | 0.24 | 0.37 |

Determination of effect between 6-(4-(tert-butyl)phenoxy)pyridin-3-amine and inhibitors of anti-apoptotic proteins in 3 cancer cell lines: Highest single agent (HSA) model was used to analyze data from combination screen. This model states that the expected effect from a combination of compounds equals to the higher effect of individual drugs at the dose in the combination (Gaddum, Pharmacology, 1940). Any additional effect over the higher single drug is considered as synergy. Table 3 shows drug candidates showing synergistic effect on human T-ALL cell line RPMI-8402, triple negative breast cancer cell line HCC1187 and ER+ breast cancer cell line MCF-7 in corresponding dose range.

**Table 3: Synergistic effect of drugs in combination with 6-(4-(tert-butyl)phenoxy)pyridin-3-amine on human cancer cell lines.**

| | | **RPMI-8402** | | **HCC1187** | | **MCF-7** | |
|---|---|---|---|---|---|---|---|
| **Drug1** | **Drug2** | Conc Drug 1 (µM) | Conc Drug 2 (µM) | Conc Drug 1 (µM) | Conc Drug 2 (µM) | Conc Drug 1 (µM) | Conc Drug 2 (µM) |
| ABT-263 (Navitoclax) | 6-(4-(tert-butyl)phenoxy)pyridin-3 -amine | 0.3-2.5 | 1.2 | / | / | / | / |
| S63845 | 6-(4-(tert-butyl)phenoxy)pyridin-3 -amine | 0.1 | 1.2-2.5 | 0.15 | 10.0 | 0.3 | 10.0 |

In the T-ALL cell line RPMI-8402 harboring a driver insertion in *NOTCH1* and loss of the ubiquitin ligase FBXW7, synergistic activities were measured under the assay conditions with ABT-263 (BCL-2 inhibitor) and S63845 (MCL-1 inhibitor).
In the TNBC cell line HCC1187 harboring a driver translocation in *NOTCH2,* synergistic activities were measured under the assay conditions with S63845 (MCL-1i). Similarly in ER+ BC cell line MCF-7 (NOTCH3 expressing cells), a combination of 6-(4-(tert-butyl)phenoxy)pyridin-3-amine with S63845 (MCL-1i) is expected to exhibit synergistic effects.

### EXAMPLE 2

### In Vivo Efficacy Evaluation of 6-(4-(tert-butyl)phenoxy)pyridin-3-amine and BCL-2 inhibitor ABT-263 (Navitoclax) in a NOTCH1 and BCL-2 positive Patient-Derived Xenograft (ACC PDX) Model Representing Human Adenoid Cystic Carcinoma in Immune-Deficient Mice

ACC PDX model was implanted into Immuno deficient mice and upon reaching a tumor volume of 150-300 mm³, animals were randomized for treatment with different agents as indicated below.

**Table 4 : Experimental details**

| | |
|---|---|
| **Animal Strain:** | Athymic Nude, Outbred Homozygous |
| **Animal Age** / **Sex:** | 6-12 weeks / Female |
| **Selected Models:** | ACC |
| **Tumor Implantation:** | SC Tumor Fragment (∼70 mg) |
| **Randomization:** | Stratified by Mean Animal TV; 150-300 mm³ |
| **Treatment Initiation:** | Day 0 |
| **Control / ROA:** | Vehicle / SC |
| **Test Agent(s)** / **ROA:** | 6-(4-(tert-butyl)phenoxy)pyridin-3-amine / SC |
| **SOC** / **ROA:** | ABT-263 / PO |
| **Data Capture Schedule:** | Twice Weekly |
| **Study Type(s)**: | gTGI |

| | |
|---|---|
| SOC : Standard of care, ROA : Route of administration | |

**Table 5: Dose and dosing schedule**

| **Group** | Treatment | Dose(mg/kg) | ROA / Schedule |
|---|---|---|---|
| 1 | Vehicle | -- | -- |
| 2 | 6-(4-(tert-butyl)phenoxy)pyridin-3-amine | 60 | SC / qdx5 to end |
| 3 | Navitoclax | 50 | PO / qdx5 to end 15 |
| 4 | 6-(4-(tert-butyl)phenoxy)pyridin-3-amine | 60 | SC / qdx5 to end |
| | Navitoclax | 50 | PO / qdx5 to end |

### Results and Conclusion :

Athymic mice transplanted with ACC PDX model were randomized into 4 treatment cohorts. Tumor bearing mice were treated with different drugs as below and according to doses as indicated in table 5 :
1. Vehicle treatment
2. 6-(4-(tert-butyl)phenoxy)pyridin-3-amine
3. ABT-263
4. 6-(4-(tert-butyl)phenoxy)pyridin-3-amine + ABT-263

Tumor volume was measured every 3-4 days. Mean tumor volume and SEM was calculated and Tumor growth inhibition was calculated using the formula TGI = 1-(TXf_{avg}-TXi_{avg})/(Cf_{avg}-Ci_{avg}), where TX is drug treatment group, f_{avg}= average tumor volume at treatment end, i_{avg} = average tumor volume upon treatment initiation, C = Vehile treatment group. As shown in figure 1 and table 6, while 6-(4-(tert-butyl)phenoxy)pyridin-3-amine and ABT-263 induces tumor growth retardation compared to vehicle treated group, combination of both drugs induces a tumor regression of ACC tumors.

**Table 6: Percentage tumor growth inhibition (TGI) in ACC PDX model treated with 6-(4-(tert-butyl)phenoxy)pyridin-3-amine and ABT-263 as a single agent and in combination.**

| **Treatment** | **Vehicle** | **6-(4-(tert-butyl)phenoxy)pyridine-3-amine** | **ABT-263** | **6-(4-(tert-butyl)phenoxy)pyridine-3-amine + ABT-263** |
|---|---|---|---|---|
| Percentange tumor growth inhibition (TGI) | | 37 % | 57 % | 108 % |

## Claims

1. A pharmaceutical combination comprising:
(a) an inhibitor of an anti-apoptotic protein;
(b) a NOTCH signaling pathway inhibitor; and optionally
(c) one or more pharmaceutically acceptable diluents, excipients or carriers.

2. A pharmaceutical combination according to claim 1, wherein said inhibitor of an anti-apoptotic protein is selected from the group consisting of a B-cell lymphoma 2 (BCL-2) inhibitor, a B-cell lymphoma XL (BCL-XL) inhibitor, a B-cell lymphoma W (BCLW) inhibitor and a Myeloid cell leukemia-1 (MCL-1) inhibitor.

3. A pharmaceutical combination according to claim 1, wherein said inhibitor of an anti-apoptotic protein is a B-cell lymphoma 2 (BCL-2) inhibitor.

4. A pharmaceutical combination according to claims 2 or 3, wherein the BCL-2 inhibitor is selected from the group consisiting of venetoclax, navitoclax, obatoclax, sabutoclax, ABT-737, PNT-2258, and S-055746, preferably is venetoclax or navitoclax.

5. A pharmaceutical combination according to claim 1, wherein said inhibitor of an anti-apoptotic protein is a Myeloid cell leukemia-1 (MCL-1) inhibitor.

6. A pharmaceutical combination according to claims 2 or 5, wherein the MCL-1 inhibitor is selected from the group consisiting of S63845, AMG-176, AMG-397, and AZD5991, preferably is S63845.

7. A pharmaceutical combination according to any one of claims 1 to 6, wherein the NOTCH signaling pathway inhibitor is selected from the group consisting of a γ-secretase inhibitor, a blocking antibody against NOTCH receptors, a blocking antibody against NOTCH ligands, and an inhibitor of NOTCH transcription complex.

8. A pharmaceutical combination according to any one of claims 1 to 6, wherein the Notch signaling pathway inhibitor is a compound of formula (I) 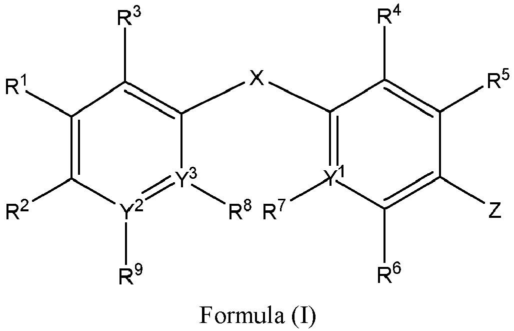 pharmaceutically-acceptable salts, hydrates, solvates, or stereoisomers thereof,
wherein X is selected from CH₂, CF₂, CHF, CO, CHOH, CHO(C₁-C₃) alkyl, NH, N(C₁-C₃ alkyl), S, SO and O;
wherein Y¹, Y², and Y³ are each independently selected from N and C;
wherein Z is NR¹⁰R¹¹, wherein R¹⁰ and R¹¹ are each independently selected from H and C₁-C₆ alkyl;
wherein R¹ is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R² is selected from C₁-C₆ alkyl, C₃-C₁₂ cycloalkyl, aryl and heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl, C(O)R¹², C₁-C₆ alkyl C(O)R¹²;
wherein R³ is selected from H, halogen, C₁-C₆ alkyl, C₁-C₆ heteroalkyl, C₃-C₁₂ cycloalkyl, C₃-C₁₂ heterocyclyl and C₁-C₆ alkoxy;
wherein R⁴, R⁵ and R⁶ are each independently selected from H, halogen, CN, C₁-C₆ alkoxy, C₁-C₆-S-alkyl, C₁-C₆ alkyl, C₁-C₆ heteroalkyl, C₃-C₁₂ cycloalkyl and C₃-C₁₂ heterocyclyl; wherein R⁷ is absent when Y¹ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y¹ is C;
wherein R⁸ is absent when Y³ is N or is selected from H, halogen, C₁-C₆ alkyl, C₁-C₆ heteroalkyl, C₃-C₁₂ cycloalkyl, C₃-C₁₂ heterocyclyl and C₁-C₆ alkoxy when Y³ is C;
wherein R⁹ is absent when Y² is N or is selected from H, halogen, C₁-C₆ alkyl, C₃-C₁₂ cycloalkyl, C₃-C₁₂ heterocyclyl, C₁-C₆ alkoxy, C₁-C₆ heteroalkyl, C₀-C₃ alkylOC₀-C₃ alkyl aryl wherein the aryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; C₀-C₃ alkylOC₀-C₃ alkyl heteroaryl wherein the heteroaryl is optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl; and C₁-C₆ alkyl substituted by aryl or heteroaryl wherein the aryl and the heteroaryl are optionally substituted by NH₂, OC₁-C₆ alkyl, C₁-C₆ alkyl, C₁-C₆ heteroalkyl, halogen, CN, C₃-C₁₂ cycloalkyl, C₃-C₁₂ heterocyclyl;
wherein R¹² is selected from H, NH₂, NHC₁-C₆ alkyl, C₁-C₆ alkyl, C₁-C₆ heteroalkyl, C₂-C₆ alkenyl, C₂-C₆ alkynyl, C₃-C₁₂ cycloalkyl and C₃-C₁₂ heterocyclyl; and optionally one or more pharmaceutically acceptable diluents, excipients or carriers.

9. A pharmaceutical combination according to any one of claims 1 to 6, wherein the NOTCH signaling pathway inhibitor is 6-(4-(tert-butyl)phenoxy)pyridin-3-amine or pharmaceutically-acceptable salts, hydrates, solvates, or stereoisomers thereof.

10. A pharmaceutical combination according to any one of claims 1 to 9, for use as a medicament.

11. A pharmaceutical combination according to any one of claims 1 to 9 for use in a method for the prevention, delay of progression or treatment of cancer in a subject.

12. A pharmaceutical combination for use according to claim 11, wherein the cancer is a solid tumor.

13. A pharmaceutical combination for use according to claim 12, wherein the solid tumor is selected from the group consisting of adenoid cystic carcinoma (ACC), breast cancer, prostate cancer, osteosarcoma, malignant glomus tumour, colorectal cancer and hepatocellular carcinoma.

14. A pharmaceutical combination for use according to claim 11, wherein the cancer is a haematological cancer.

15. A pharmaceutical combination for use according to claim 14, wherein the haematological cancer is selected from the group consisting of acute lymphoblastic leukemia, T-cell acute lymphoblastic leukemia or T-cell lymphoblastic lymphoma, acute myeloid leukemia, chronic lymphocytic leukemia, chronic myelocytic leukemia, Hodgkin lymphoma, non-Hodgkin lymphoma, follicular lymphoma, diffuse large B cell lymphoma, Burkitt lymphoma, marginal zone B-cell lymphoma, splenic marginal zone lymphoma, mantle cell lymphoma, peripheral T-cell lymphoma, anaplastic large cell lymphoma, CNS lymphoma, and multiple myeloma.

16. A pharmaceutical combination for use according to claim 11, wherein the cancer is selected from the group consisting of T-cell acute lymphoblastic leukemia, breast cancer and adenoid cystic carcinoma (ACC), and is preferably T-cell acute lymphoblastic leukemia and breast cancer, more preferably T-cell acute lymphoblastic leukemia and triple negative breast cancer (TNBC).
